# EUROPEAN PATENT APPLICATION

(11) **EP 4 049 664 A1**
(43) Date of publication of application: **31.08.2022**
(21) Application number: 20879612.8
(22) Date of filing: 26.10.2020
(51) Int. Cl.: A61K 31/7105, A61K 31/713, A61K 47/22, A61K 47/42, A61P 1/18, A61P 35/00, A61P 35/04, C07K 7/00, C07K 14/00, C12N 15/113

(54) **NUCLEIC ACID DELIVERY ENHANCER**

(30) Priority: 25.10.2019 JP 2019194646
(71) Applicant: National University Corporation Kochi University, Kochi-shi, Kochi 780-8520 (JP); Tokyo University of Science Foundation, Tokyo 162-8601 (JP)
(72) Inventor: TANIUCHI Keisuke, Kochi-shi, Kochi 780-8520 (JP); WADA Takeshi, Tokyo 162-8601 (JP); HARA Rintaro, Tokyo 162-8601 (JP); SATO Kazuki, Tokyo 162-8601 (JP); TAKAGI Kazunori, Tokyo 162-8601 (JP)
(74) Representative: J A Kemp LLP
(86) International application number: PCT/JP2020/040071
(87) International publication number: WO 2021/080020

(57) **Abstract**

A novel anti-tumor agent capable of delivering siRNA or shRNA specifically to pancreatic cancer cells and suppressing tumor growth, invasion, and metastasis of pancreatic cancer is provided. The present invention provides a nucleic acid delivery enhancer for delivering siRNA or shRNA into cells, which consists of a folic acid-cationic oligopeptide complex. The present invention also provides an anti-tumor agent comprising siRNA or shRNA capable of binding to mRNA or snoRNA expressed in pancreatic cancer cells to inhibit its expression and a folic acid-cationic oligopeptide complex. The siRNA is capable of binding to RNA selected from the group consisting of, for example, SNORA18 snoRNA, NUP85 mRNA, WASF2 mRNA, and SNORA22 snoRNA.

SEQUENCE LISTING

<110>  National University Corporation Kochi University
       Tokyo University of Science Foundation

<120>  Agent for Promoting Nucleic Acid Delivery

<130>  PH-8588-PCT

<150>  JP 2019-194646
<151>  2019-10-25

<160>  28

<170>  PatentIn version 3.5

<210>  1
<211>  21
<212>  DNA
<213>  Artificial

<220>
<223>  siRNA sense strand

<220>
<223>  DNA/RNA binding molecule

<400>  1
gucaucacac ugaauaccat t                                                21


<210>  2
<211>  21
<212>  DNA
<213>  Artificial

<220>
<223>  siRNA antisense strand

<220>
<223>  DNA/RNA binding molecule

<400>  2
ugguauucag ugugaugact t                                                21


<210>  3
<211>  21
<212>  RNA
<213>  Artificial

<220>
<223>  control siRNA

<400>  3
uucuccgaac gugucacgua u                                                21


<210>  4
<211>  21
<212>  RNA
<213>  Artificial

<220>
<223>  control siRNA

<400>  4
auacgugaca cguucggaga a                                                21


<210>  5
<211>  21
<212>  RNA
<213>  Artificial

<220>
<223>  SNORA18 siRNA sense strand

<400>  5
uuuacuuuac ucacaggacu a                                                21


<210>  6
<211>  21
<212>  RNA
<213>  Artificial

<220>
<223>  SNORA18 siRNA antisense strand

<400>  6
uaguccugug aguaaaguaa a                                                21


<210>  7
<211>  21
<212>  RNA
<213>  Artificial

<220>
<223>  NUP85 siRNA sense strand

<400>  7
cagcggcaga ugacugaaca a                                                21


<210>  8
<211>  21
<212>  RNA
<213>  Artificial

<220>
<223>  NUP85 siRNA antisense strand

<400>  8
uuguucaguc aucugccgcu g                                                21


<210>  9
<211>  21
<212>  RNA
<213>  Artificial

<220>
<223>  WASF2 siRNA sense strand

<400>  9
uaggauuaga ucauuagcuc a                                                21


<210>  10
<211>  21
<212>  RNA
<213>  Artificial

<220>
<223>  WASF2 siRNA antisense strand

<400>  10
ugagcuaaug aucuaauccu a                                                21


<210>  11
<211>  21
<212>  RNA
<213>  Artificial

<220>
<223>  SNORA22 siRNA sense strand

<400>  11
cuuggcuuug acccugugcu a                                                21


<210>  12
<211>  21
<212>  RNA
<213>  Artificial

<220>
<223>  SNORA22 siRNA antisense strand

<400>  12
uagcacaggg ucaaagccaa g                                                21


<210>  13
<211>  20
<212>  DNA
<213>  Artificial

<220>
<223>  primer

<400>  13
ccctcttggt agcttcgttc                                                  20


<210>  14
<211>  23
<212>  DNA
<213>  Artificial

<220>
<223>  primer

<400>  14
cgcaggtatg aaataagact gag                                              23

<210>  15
<211>  21
<212>  DNA
<213>  Artificial

<220>
<223>  primer

<400>  15
gctcctccag tcacacaacc t                                                21


<210>  16
<211>  24
<212>  DNA
<213>  Artificial

<220>
<223>  primer

<400>  16
ctcggggcag taatcaaagt aatc                                             24


<210>  17
<211>  21
<212>  DNA
<213>  Artificial

<220>
<223>  primer

<400>  17
gtgccagctt ggacagattg a                                                21


<210>  18
<211>  20
<212>  DNA
<213>  Artificial

<220>
<223>  primer

<400>  18
ggacacggtg ggaatgctta                                                  20


<210>  19
<211>  20
<212>  DNA
<213>  Artificial

<220>
<223>  primer

<400>  19
gtgcagggag aggaatcaat                                                  20


<210>  20
<211>  20

<212>  DNA
<213>  Artificial

<220>
<223>  primer

<400>  20
gcatgtacga aagctccaga                                                    20


<210>  21
<211>  21
<212>  DNA
<213>  Artificial

<220>
<223>  SNORA18 modified siRNA sense strand

<220>
<221>  modified_base
<222>  (1)..(1), (6)..(6), (18)..(18)
<223>  um

<220>
<221>  modified_base
<222>  (8)..(8), (10)..(10)
<223>  cm

<220>
<223>  DNA/RNA binding molecule

<400>  21
uacuuuacuc acaggacuat t                                                  21


<210>  22
<211>  21
<212>  RNA
<213>  Artificial

<220>
<223>  SNORA18 modified siRNA antisense strand

<220>
<221>  modified_base
<222>  (1)..(1), (13)..(13), (18)..(18)
<223>  um

<400>  22
uaguccugug aguaaaguaa a                                                  21


<210>  23
<211>  21
<212>  DNA
<213>  Artificial

<220>
<223>  NUP85 modified siRNA sense strand

<220>
<221>  modified_base
<222>  (5)..(5), (17)..(17)

<223>  cm

<220>
<223>  DNA/RNA binding molecule

<400>  23
gcggcagaug acugaacaat t                                                21


<210>  24
<211>  21
<212>  RNA
<213>  Artificial

<220>
<223>  NUP85 modified siRNA antisense strand

<220>
<221>  modified_base
<222>  (6)..(6), (10)..(10)
<223>  cm

<400>  24
uuguucaguc aucugccgcu g                                                21


<210>  25
<211>  21
<212>  DNA
<213>  Artificial

<220>
<223>  WASF2 modified siRNA sense strand

<220>
<221>  modified_base
<222>  (5)..(5), (13)..(13)
<223>  um

<220>
<221>  modified_base
<222>  (10)..(10), (18)..(18)
<223>  cm

<220>
<223>  DNA/RNA binding molecule

<400>  25
ggauuagauc auuagcucat t                                                21


<210>  26
<211>  21
<212>  RNA
<213>  Artificial

<220>
<223>  WASF2 modified siRNA antisense strand

<220>
<221>  modified_base
<222>  (6)..(6), (14)..(14), (20)..(20)
<223>  um

<400>  26
ugagcuaaug aucuaauccu a                                                21

<210>  27
<211>  21
<212>  DNA
<213>  Artificial

<220>
<223>  SNORA22 modified siRNA sense strand

<220>
<221>  modified_base
<222>  (18)..(18)
<223>  um

<220>
<223>  DNA/RNA binding molecule

<400>  27
uggcuuugac ccugugcuat t                                                21

<210>  28
<211>  21
<212>  RNA
<213>  Artificial

<220>
<223>  SNORA22 modified siRNA antisense strand

<220>
<221>  modified_base
<222>  (1)..(1)
<223>  um

<220>
<221>  modified_base
<222>  (4)..(4), (6)..(6), (12)..(12), (18)..(18)
<223>  cm

<400>  28
uagcacaggg ucaaagccaa g                                                21

## Description

### Technical Field

The present invention relates to a nucleic acid delivery enhancer for delivering nucleic acid molecules such as siRNA into cells. The present invention also relates to a drug that can be used in treatment of diseases or the like by utilizing RNA interference (RNAi). Particularly, the present invention relates to a nucleic acid formulation that has an anti-tumor effect on pancreatic cancer and can effectively suppress tumor growth, invasion, and metastasis of pancreatic cancer, and a pharmaceutical composition comprising the same.

### Background Art

Pancreatic cancer is said to have the worst prognosis among cancers. The reasons for this are that the pancreas is a retroperitoneal organ, making early detection difficult and that the extremely high motility of pancreatic cancer cells results in a strong tendency for peritoneal invasion and metastasis to blood vessels, gastrointestinal tract, nerves, and the like.

In investigating mechanisms of invasion and metastasis of pancreatic cancer, the present inventors have previously found that insulin-like growth factor 2 mRNA-binding protein 3 (IGF2BP3), which is normally found in the nucleolus and is known to bind to the 5' untranslated region of insulin-like growth factor II mRNA and inhibit translation of the insulin-like growth factor II, is present in the cell membrane protrusion in pancreatic cancer cells, and that various mRNAs bind to the IGF2BP3 and accumulate in the cell membrane protrusion. They have also reported that inhibition of these mRNAs by RNA interference (RNAi) effectively suppresses invasion and metastasis of pancreatic cancer cells (Patent Literature 1).

siRNA (small interfering RNA), a representative nucleic acid medicine utilizing RNA interference (RNAi), which has recently attracted attention as a new therapeutic agent, is a small molecule double-stranded RNA generally consisting of 21 to 23 base pairs. However, siRNA has been problematic in terms of delivery into cells due to its high anionic nature and low cell-membrane permeability because of its structure. To address this problem, the present inventors have previously produced a siRNA-folic acid-polyethylene glycol (PEG)-chitosan oligosaccharide lactate (COL) nanoparticle complex and confirmed enhancing effects of these nanoparticles on the uptake of siRNA into cells. They have also reported that siRNA against mRNA having the IGF2BP3 binding ability taken up by pancreatic cancer cells suppresses invasion and metastasis of pancreatic cancer (Non Patent Literature 1).

On the other hand, the present inventors have found that a cationic oligopeptide having an amino group or a guanidino group on a side chain is useful for the delivery of double-stranded RNA into a cell (Patent Literature 2 and Non Patent Literature 2). To deliver RNAi molecules, the use of complexes comprising vitamin E and cationic sugar has also been investigated (Non Patent Literatures 3 and 4).

### Citation List

### Patent Literature

Patent Literature 1: International Publication No. WO 2016/002844
Patent Literature 2: International Publication No. WO 2014/148620

### Non Patent Literature

Non Patent Literature 1: Oncotarget, 2019, Vol. 10, No. 30, pp. 2869-2886
Non Patent Literature 2: Bioorganic & Medicinal Chemistry 21 (2013) 1717-1723
Non Patent Literature 3: Bioorganic & Medicinal Chemistry 22 (2014) 1394-1403
Non Patent Literature 4: Bioorganic & Medicinal Chemistry Letters 25 (2015) 815-819

### Summary of Invention

### Technical Problem

As described above, how to achieve the delivery of RNAi molecules such as siRNA to cells as a nucleic acid medicine is a critical problem for enhancing the practicality of the nucleic acid medicine. There are still many problems to be solved, such as how to achieve not only just delivery, but also specific delivery to target cells, and how to keep the effects of the nucleic acid medicine unperturbed after taken up into cells.

### Solution to Problem

As a result of an extensive study in the light of the above problems, the present inventors have found that siRNA and shRNA can be specifically delivered into cells having a folic acid receptor, by using a complex of folic acid and a cationic oligopeptide. Using this approach, they have also found a new therapeutic strategy for pancreatic cancer that can suppress tumor growth, invasion, and metastasis by targeting pancreatic cancer cells and effectively allowing siRNA or shRNA to be taken up by the target pancreatic cancer cells to knock down the expression of specific RNAs, thus completing the present invention.

The present invention provides as follows:
1. A delivery enhancer of siRNA or shRNA consisting of a folic acid-cationic oligopeptide complex, wherein the cationic oligopeptide comprises a cationic oligopeptide moiety consisting of 8 to 40 amino acids, comprising at least two contiguous amino acid residues of the following formula (I), and optionally comprising another non-contiguous amino acid residue other than the contiguous amino acid residues of the following formula (I): 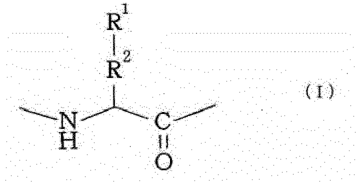 wherein R¹ is a group H₃N⁺-CH₂- or a group represented by formula (II); R² is absent or an alkylene group having 1 to 3 carbon atoms when R¹ is the group H₃N⁺-CH₂-, or R² is an alkylene group having 1 to 4 carbon atoms when R¹ is a group represented by formula (II); and in one cationic oligopeptide, all R¹ are the same and all R² are the same: 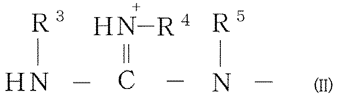 wherein R³, R⁴, and R⁵ are the same or different and are each a hydrogen atom or a methyl group.
2. The delivery enhancer according to 1 above, wherein the cationic oligopeptide moiety consists of 8 to 12 amino acids.
3. The delivery enhancer according to 1 or 2 above, wherein the cationic oligopeptide moiety is a homomultimer of L-2,3-diaminopropionic acid (Dap), L-2,4-diaminobutyric acid (Dab), L-ornithine (Orn), L-lysine (Lys), L-2-amino-3-guanidinopropionic acid (Agp), L-2-amino-4-guanidinobutyric acid (Agb), or L-arginine (Arg).
4. The delivery enhancer according to any of 1 to 3 above, wherein the cationic oligopeptide has as substructure an octamer of diaminobutyric acid having the following structure. 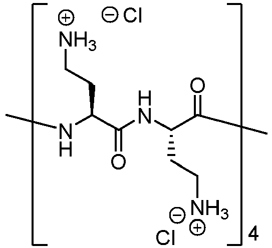
5. The delivery enhancer according to any of 1 to 4 above, wherein the folic acid is linked to the N-terminus, the C-terminus, or a side chain of the cationic oligopeptide via a linker or no linker.
6. The delivery enhancer according to 5 above, wherein the folic acid-cationic oligopeptide complex is linked via a linker, the linker being a peptide linker.
7. The delivery enhancer according to 6 above, wherein the peptide linker is a peptide consisting of 1 to 4 glycine residues.
8. The delivery enhancer according to 4 above, wherein the folic acid-cationic oligopeptide complex is Fol-Dab8A and/or Fol-Dab8B having the following structure. 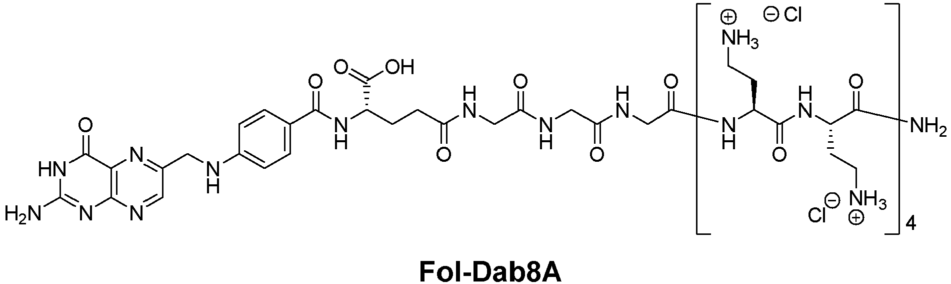 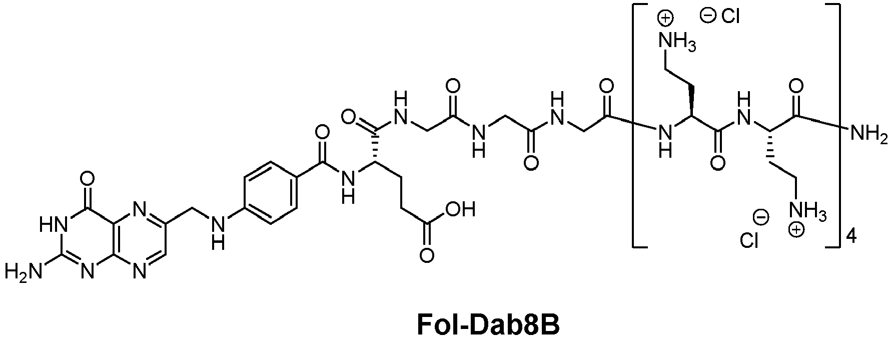
9. An anti-tumor agent comprising: siRNA or shRNA capable of binding to mRNA or snoRNA expressed in pancreatic cancer cells to inhibit its expression; and the delivery enhancer according to any of 1 to 8 above.
10. The anti-tumor agent according to 9 above, wherein the mRNA or snoRNA expressed in pancreatic cancer cells binds to insulin-like growth factor 2 mRNA-binding protein 3 (IGF2BP3).
11. The anti-tumor agent according to 9 or 10 above, wherein the mRNA or snoRNA expressed in pancreatic cancer cells is selected from the group consisting of SNORA18 snoRNA, NUP85 mRNA, WASF2 mRNA, and SNORA22 snoRNA.
12. The anti-tumor agent according to any of 9 to 11 above, comprising 0.5 to 10 equivalents of the folic acid-cationic oligopeptide complex relative to the siRNA or shRNA.
13. The anti-tumor agent according to any of 9 to 12 above, wherein the siRNA is an RNA-RNA duplex.
14. The anti-tumor agent according to any of 9 to 13 above, wherein the siRNA or shRNA comprises a modified base, a modified sugar, and/or an altered internucleoside bond.
15. The anti-tumor agent according to 14 above, wherein the modification of the modified sugar is 2'-OMe modification.
16. The anti-tumor agent according to 14 or 15 above, wherein the altered internucleoside bond is phosphorothioate bond.
17. A pharmaceutical composition comprising the anti-tumor agent according to any of 9 to 16 above.
18. A combined formulation comprising:
   (a) a formulation comprising siRNA or shRNA capable of binding to mRNA or snoRNA expressed in pancreatic cancer cells to inhibit its expression; and
   (b) a formulation comprising a folic acid-cationic oligopeptide complex comprising a cationic oligopeptide moiety consisting of 8 to 40 amino acids, comprising at least two contiguous amino acid residues of the following formula (I), and optionally comprising another non-contiguous amino acid residue other than the contiguous amino acid residues of the following formula (I): 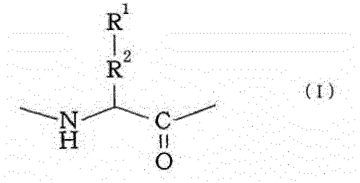 wherein R¹ is a group H₃N⁺-CH₂- or a group represented by formula (II); R² is absent or an alkylene group having 1 to 3 carbon atoms when R¹ is the group H₃N⁺-CH₂-, or R² is an alkylene group having 1 to 4 carbon atoms when R¹ is a group represented by formula (II); and in one cationic oligopeptide, all R¹ are the same and all R² are the same: 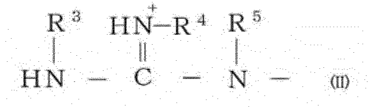 wherein R³, R⁴, and R⁵ are the same or different and are each a hydrogen atom or a methyl group.
19. A pharmaceutical kit for treating pancreatic cancer comprising:
   (a) siRNA or shRNA capable of binding to mRNA or snoRNA expressed in pancreatic cancer cells to inhibit its expression; and
   (b) a folic acid-cationic oligopeptide complex comprising a cationic oligopeptide moiety consisting of 8 to 40 amino acids, comprising at least two contiguous amino acid residues of the following formula (I), and optionally comprising another non-contiguous amino acid residue other than the contiguous amino acid residues of the following formula (I): 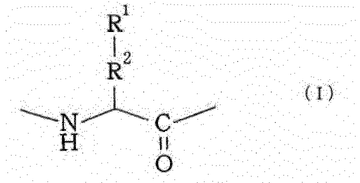 wherein R¹ is a group H₃N⁺-CH₂- or a group represented by formula (II); R² is absent or an alkylene group having 1 to 3 carbon atoms when R¹ is the group H₃N⁺-CH₂-, or R² is an alkylene group having 1 to 4 carbon atoms when R¹ is a group represented by formula (II); and in one cationic oligopeptide, all R¹ are the same and all R² are the same: 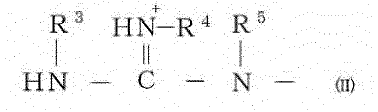 wherein R³, R⁴, and R⁵ are the same or different and are each a hydrogen atom or a methyl group.

The present specification incorporates the contents disclosed in JP Patent Application No. 2019-194646, which is the basis for the priority of the present application.

### Advantageous Effects of Invention

According to the present invention, a nucleic acid formulation capable of effectively delivering siRNA or shRNA specifically to pancreatic cancer cells and suppressing tumor growth, invasion, and metastasis of pancreatic cancer is provided.

### Brief Description of Drawings

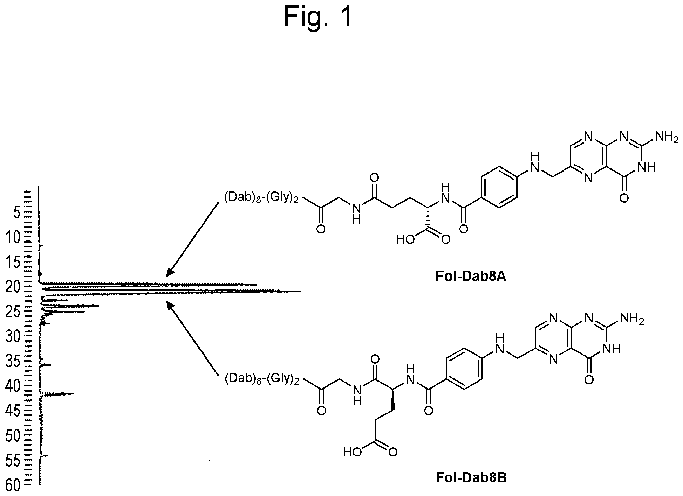

[Figure 1] Figure 1 shows HPLC analysis results of a crude product containing Fol-Dab8A and Fol-Dab8B.

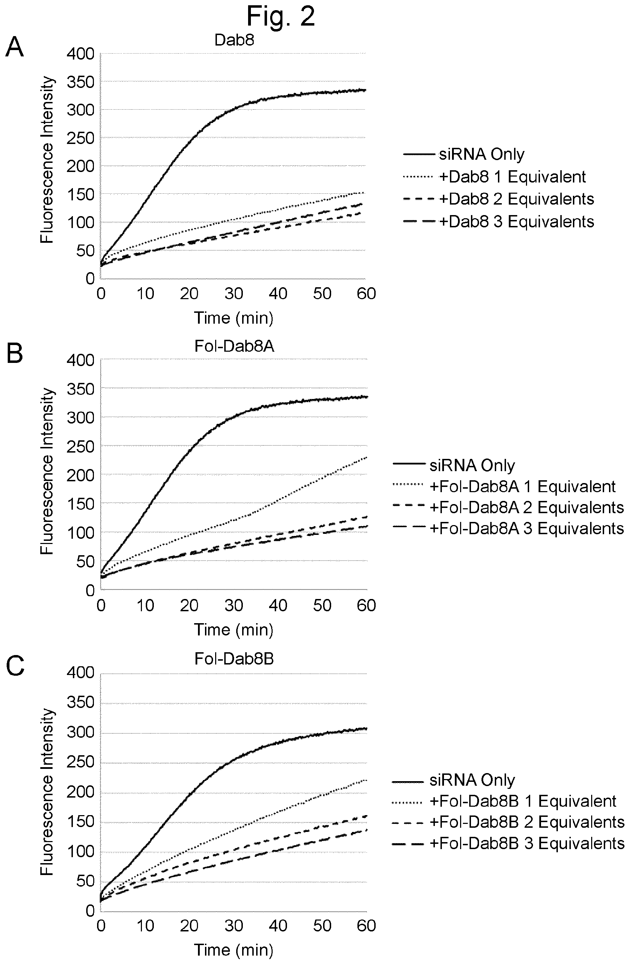

[Figure 2] Figure 2 illustrates effects of 1 to 3 equivalents of Dab8, Fol-Dab8A, and Fol-Dab8B on RNase A resistance of siRNA by changes in fluorescence intensity through fluorescence resonance energy transfer (FRET).

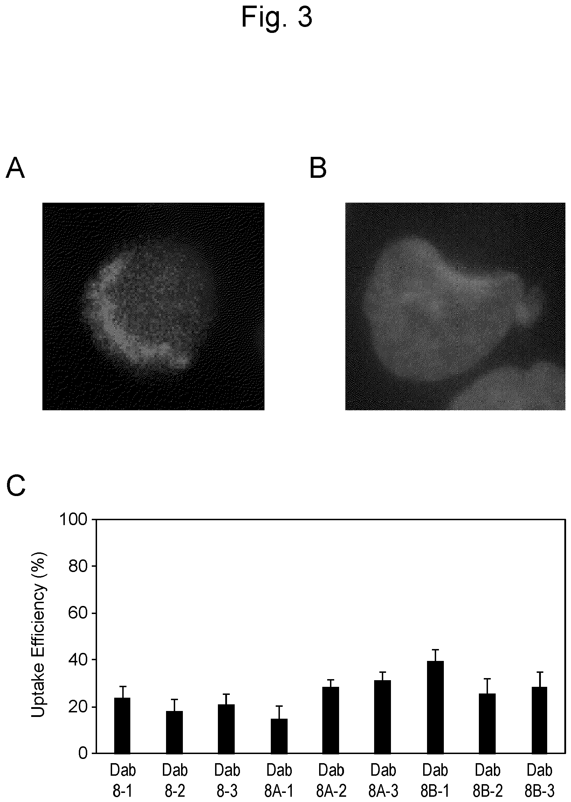

[Figure 3] Figure 3A shows a confocal microscope image illustrating the uptake of Alexa488-labeled siRNA into S2-013 pancreatic cancer cells in the presence of a folic acid-cationic oligopeptide complex. Figure 3B shows a confocal microscope image illustrating the uptake of Alexa488-labeled siRNA into S2-013 pancreatic cancer cells in the absence of a folic acid-cationic oligopeptide complex. Figure 3C illustrates the uptake efficiency of siRNA into S2-013 pancreatic cancer cells in the presence of a cationic oligopeptide or a folic acid-cationic oligopeptide complex. Results are shown as mean and standard error. "Dab8-1" indicates that the cationic oligopeptide Dab8 was added in 1 equivalent, and "Fol-Dab8B-3" indicates that the folic acid-cationic oligopeptide complex Dab8B was added in 3 equivalents.

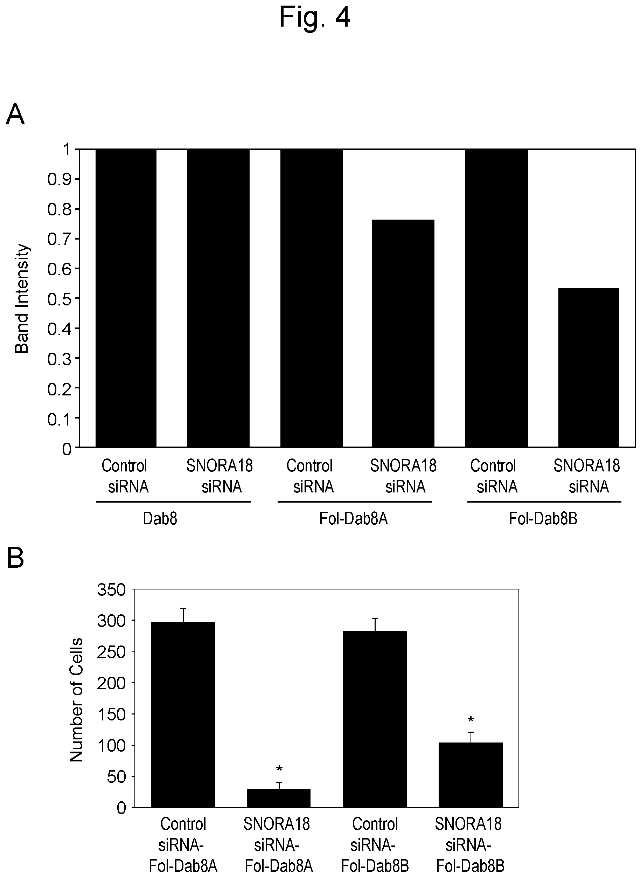

[Figure 4] Figure 4A illustrates suppressing effects on the expression of SNORA18, provided by using a scrambled control siRNA or SNORA18 siRNA in combination with a cationic oligopeptide or a folic acid-cationic oligopeptide complex. The data show bands amplified by RT-PCR as relative intensities with a band intensity of 1 in the combination of control siRNA and Dab8. Figure 4B illustrates the number of infiltrating cells in Matrigel assay using a scrambled control siRNA or SNORA18 siRNA in combination with folic acid-cationic peptide complexes (Fol-Dab8A and Fol-Dab8B).

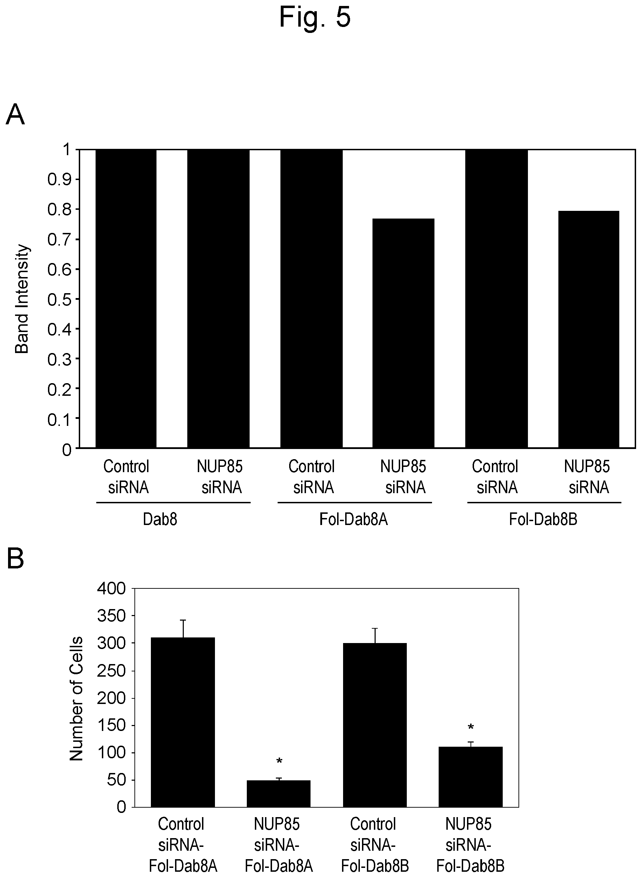

[Figure 5] Figure 5A illustrates suppressing effects on the expression of NUP85, provided by using a scrambled control siRNA or NUP85 siRNA in combination with a cationic oligopeptide or a folic acid-cationic oligopeptide complex. The data show bands amplified by RT-PCR as relative intensities with a band intensity of 1 in the combination of control siRNA and Dab8. Figure 5B illustrates the number of infiltrating cells in Matrigel assay using a scrambled control siRNA or NUP85 siRNA in combination with folic acid-cationic peptide complexes (Fol-Dab8A and Fol-Dab8B).

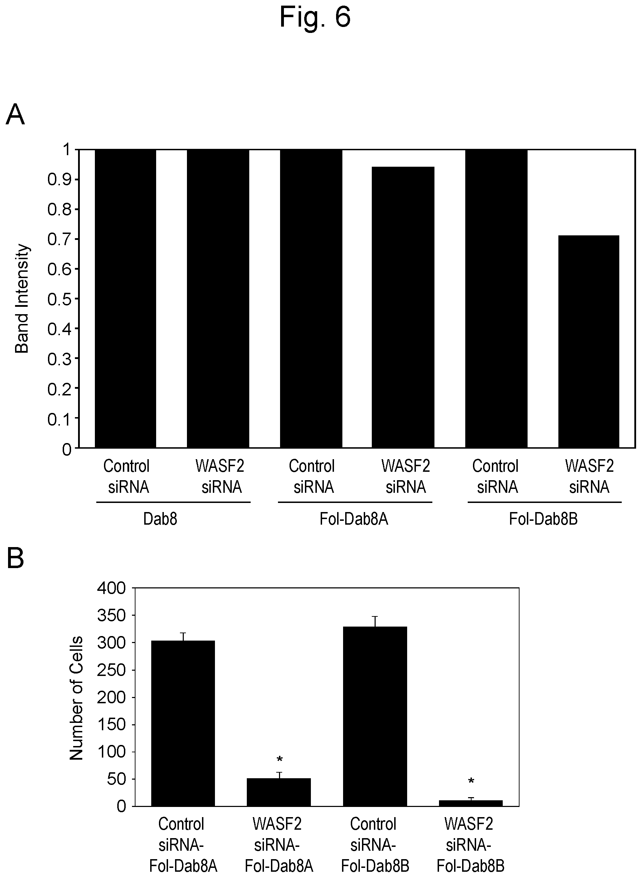

[Figure 6] Figure 6A illustrates suppressing effects on the expression of WASF2, provided by using a scrambled control siRNA or WASF2 siRNA in combination with a cationic oligopeptide or a folic acid-cationic oligopeptide complex. The data show bands amplified by RT-PCR as relative intensities with a band intensity of 1 in the combination of control siRNA and Dab8. Figure 6B illustrates the number of infiltrating cells in Matrigel assay using a scrambled control siRNA or WASF2 siRNA in combination with folic acid-cationic peptide complexes (Fol-Dab8A and Fol-Dab8B).

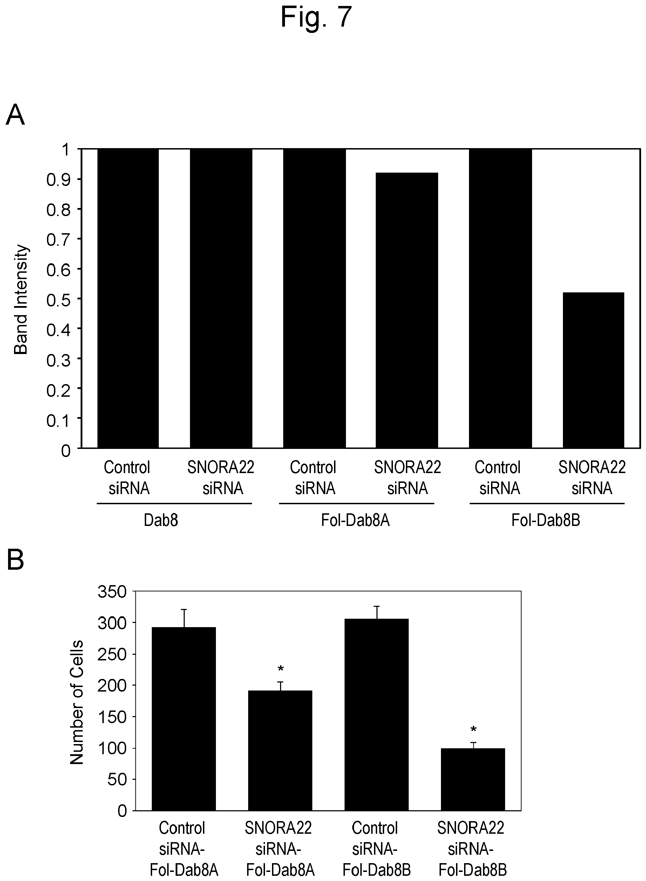

[Figure 7] Figure 7A illustrates suppressing effects on the expression of SNORA22, provided by using a scrambled control siRNA or SNORA22 siRNA in combination with a cationic oligopeptide or a folic acid-cationic oligopeptide complex. The data show bands amplified by RT-PCR as relative intensities with a band intensity of 1 in the combination of control siRNA and Dab8. Figure 7B illustrates the number of infiltrating cells in Matrigel assay using a scrambled control siRNA or SNORA22 siRNA in combination with folic acid-cationic peptide complexes (Fol-Dab8A and Fol-Dab8B).

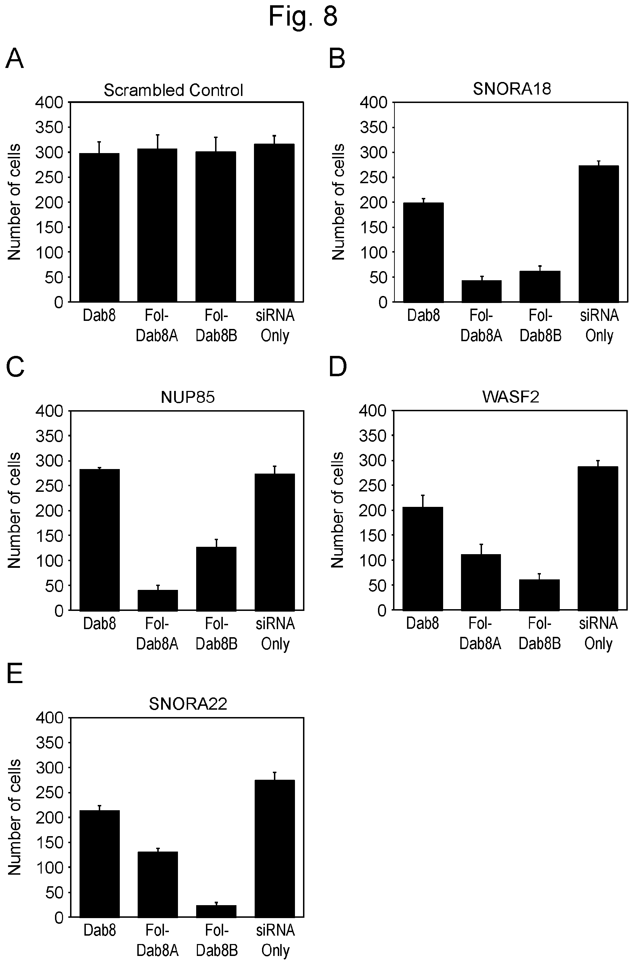

[Figure 8] Figure 8 illustrates the number of infiltrating cells in Matrigel assay using a scrambled control siRNA (A), SNORA18 siRNA (B), NUP85 siRNA (C), WASF2 siRNA (D), or SNORA22 siRNA (E) in combination with a cationic oligopeptide (Dab8) or folic acid-cationic peptide complexes (Fol-Dab8A and Fol-Dab8B).

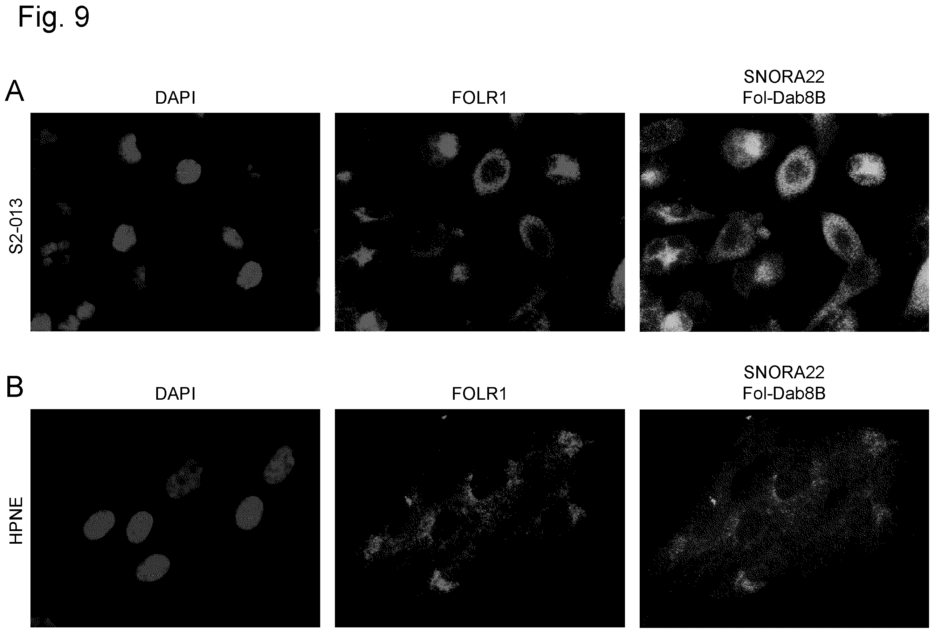

[Figure 9] Figure 9 is confocal microscope images illustrating staining of folate receptor (FOLR1) and SNORA22 siRNA in S2-013 pancreatic cancer cells (A) and HPNE normal pancreatic duct epithelial cells (B) incubated overnight with Alexa488-labeled SNORA22 siRNA and a folic acid-cationic oligopeptide complex (Fol-Dab8B, 2 equivalents) added to a culture solution.

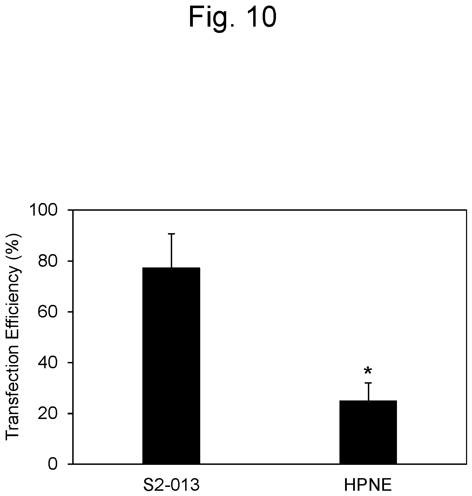

[Figure 10] Figure 10 illustrates the incorporation efficiency (%) of SNORA22 siRNA into S2-013 pancreatic cancer cells and HPNE normal pancreatic duct epithelial cells in the presence of a folic acid-cationic oligopeptide complex (Fol-Dab8B, 2 equivalents). Results are shown as mean and standard error. ^{∗} denotes a significant difference at P < 0.05.

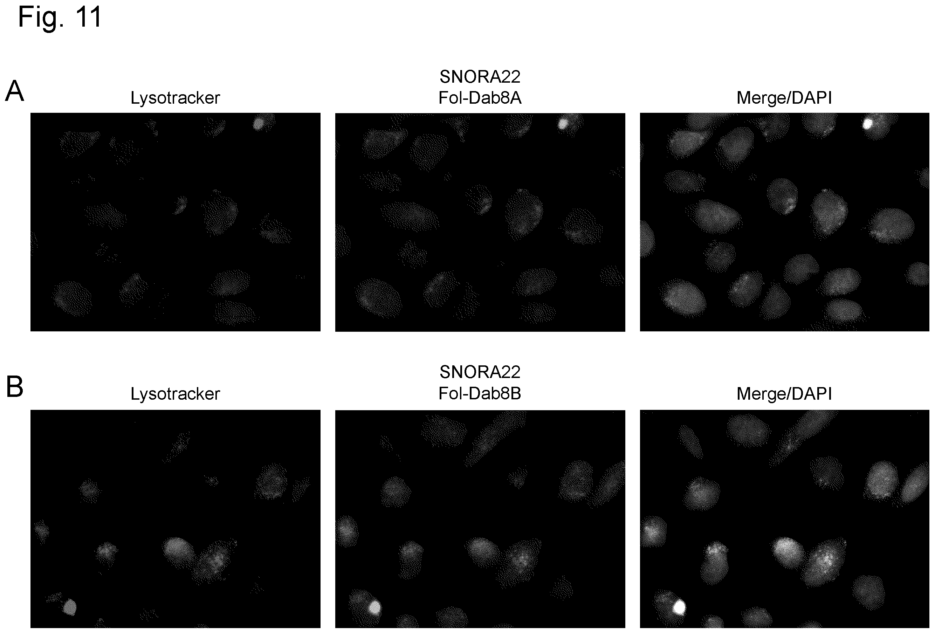

[Figure 11] Figure 11 is confocal microscope images illustrating staining of lysosome (LysoTracker) and SNORA22 siRNA in S2-013 pancreatic cancer cells incubated overnight with Alexa488-labeled SNORA22 siRNA and a folic acid-cationic oligopeptide complex (Fol-Dab8B (A) or Fol-Dab8B (B), 2 equivalents) added to a culture solution. Merge/DAPI is an image of Alexa488 staining and DAPI staining combined.

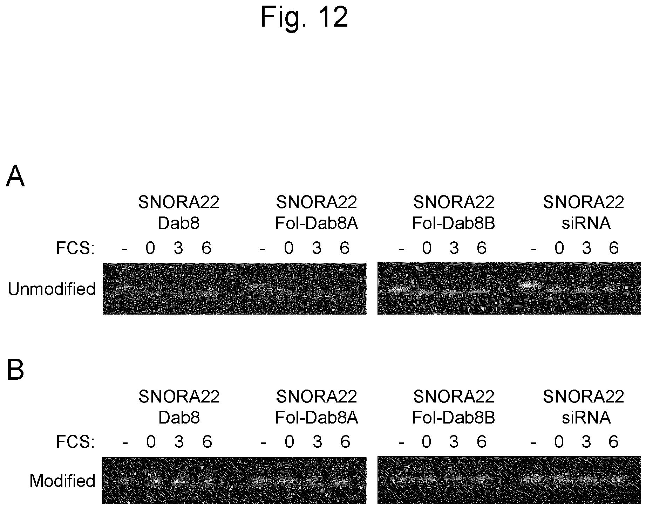

[Figure 12] Figure 12 illustrates the stability of siRNA immediately (0), 3 hours (3), or 6 hours (6) after addition of chemically-unmodified SNORA22 siRNA (A) or chemically-modified SNORA22 siRNA (B) mixed with a cationic oligopeptide (Dab8) or a folic acid-cationic oligopeptide complex (Fol-Dab8A or Fol-Dab8B) to serum (10% FCS/PBS), shown by results of SDS-PAGE using a non-reducing gel. -: no FCS (PBS only); and control: each SNORA22 siRNA added alone.

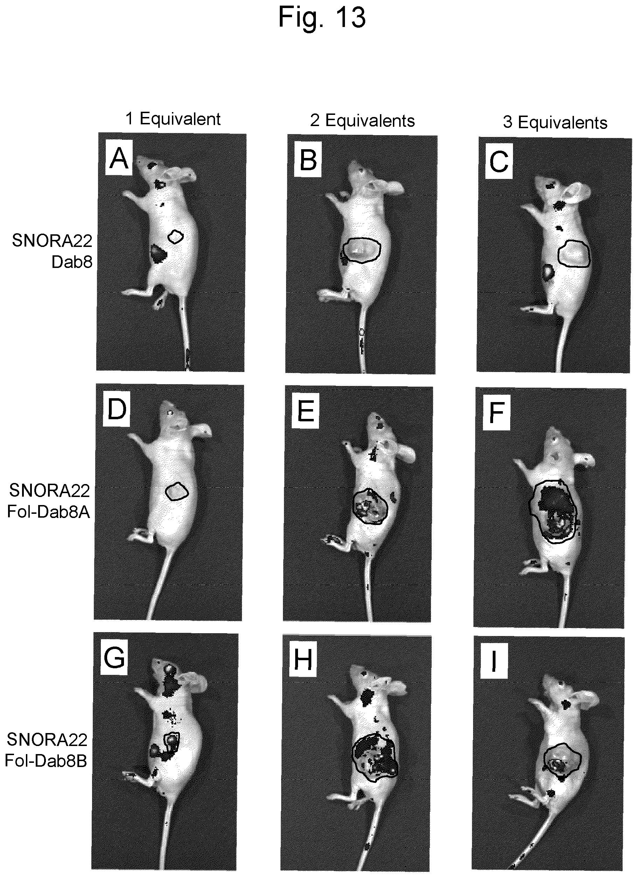

[Figure 13] Figure 13 shows results of delivery of chemically-modified SNORA22 siRNA to pancreatic cancer tissue of mice carrying human pancreatic cancer organoids derived from S2-013 pancreatic cancer cells, imaged using an *in vivo* imager. Figures 13A to 13C illustrate cases where 1 to 3 equivalents of Dab8 were added to SNORA22 siRNA; Figures 13D to 13F illustrate cases where 1 to 3 equivalents of Fol-Dab8A were added to SNORA22 siRNA; and Figures 13G to 131 illustrate cases where 1 to 3 equivalents of Fol-Dab8B were added to SNORA22 siRNA.

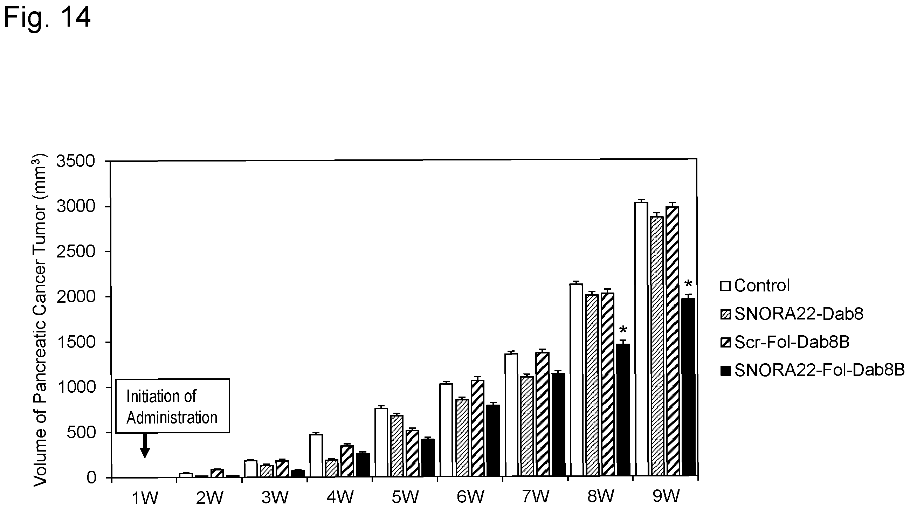

[Figure 14] Figure 14 illustrates anti-tumor effects of co-administration of SNORA22 siRNA and a folic acid-cationic oligopeptide complex on pancreatic cancer tumors in mice carrying human pancreatic cancer organoids derived from S2-013 pancreatic cancer cells. Control: non-administered control group; SNORA22-Dab8: group of SNORA22 siRNA administered with a folic acid-cationic oligopeptide complex; Scr-Fol-Dab8B: group of scrambled control siRNA administered; and SNORA22-Fol-Dab8B: group of SNORA22 siRNA administered with a folic acid-cationic oligopeptide complex. ^{∗} denotes a significant difference at P < 0.05.

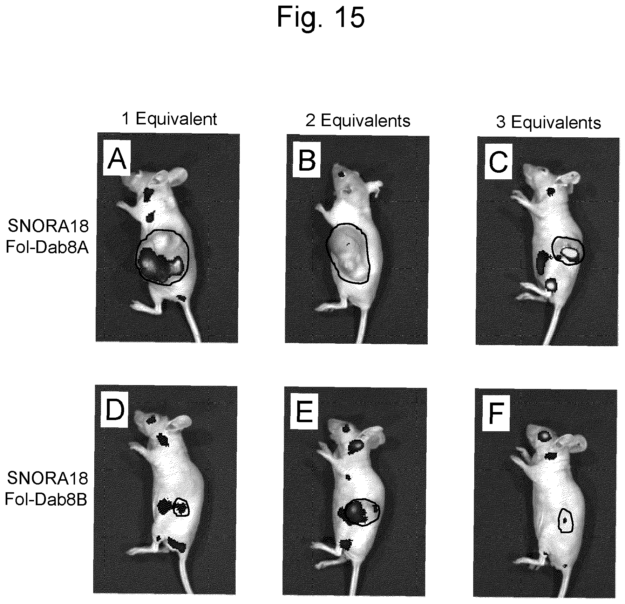

[Figure 15] Figure 15 shows results of delivery of chemically-modified SNORA18 siRNA to pancreatic cancer tissue of mice carrying human pancreatic cancer organoids derived from S2-013 pancreatic cancer cells, imaged using an *in vivo* imager. Figures 15A to 15C illustrate cases where 1 to 3 equivalents of Fol-Dab8A were added to SNORA18 siRNA; and Figures 15D to 15F illustrate cases where 1 to 3 equivalents of Fol-Dab8B were added to SNORA18 siRNA.

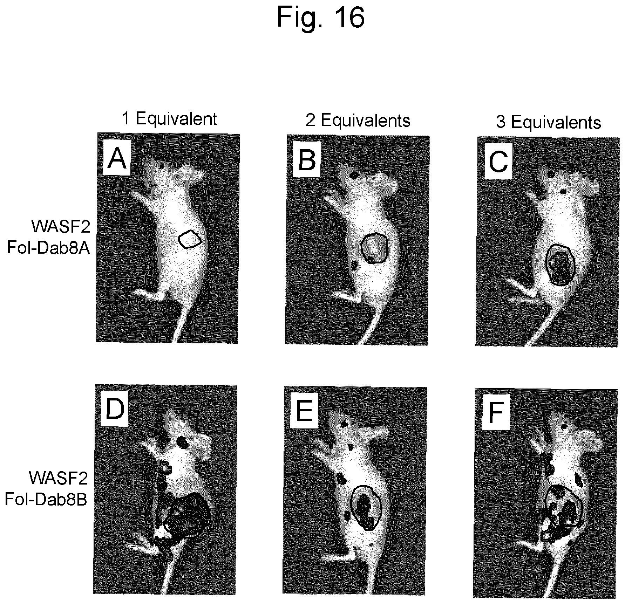

[Figure 16] Figure 16 shows results of delivery of chemically-modified WASF2 siRNA to pancreatic cancer tissue of mice carrying human pancreatic cancer organoids derived from S2-013 pancreatic cancer cells, imaged using an *in vivo* imager. Figures 16A to 16C illustrate cases where 1 to 3 equivalents of Fol-Dab8A were added to WASF2 siRNA; and Figures 16D to 16F illustrate cases where 1 to 3 equivalents of Fol-Dab8B were added to WASF2 siRNA.

### Description of Embodiments

Hereinafter, the present invention is described in more detail with reference to Examples. However, the technical scope of the present invention is not limited to these Examples.

### <Nucleic Acid Delivery Enhancer>

The present invention provides a nucleic acid delivery enhancer consisting of a folic acid-cationic oligopeptide complex.

### <Folic Acid>

Folic acid is a type of water-soluble vitamin B group and has the following structure. It has been reported that folate receptors are expressed at high concentrations on the surface of tumor cells. The present inventors have previously reported the intracellular delivery of siRNA using a complex containing folic acid in pancreatic cancer cell line S2-013, in which a folate receptor is expressed (Oncotarget, 2019, Vol. 10, No. 30, pp. 2869-2886).

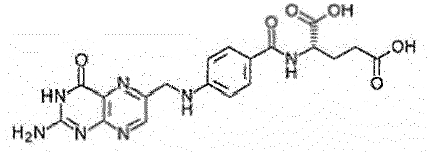

The folic acid used to produce a complex with the cationic oligopeptide in the present invention may be folic acid having the structure above, or it may be a folate or a folate derivative that retains the binding ability to the cationic oligopeptide and the folate receptor.

For example, as is apparent from the structure above, there are two carboxyl groups, an amino group, an imino group, and the like in the folic acid molecule, and substituents known in the art can also be added to these functional groups not involved in the binding to the cationic oligopeptide, and a labeled compound can also be attached to the folic acid.

### <Cationic Oligopeptide>

As a cationic oligonucleotide usable in the present invention, those disclosed in International Publication No. WO 2014/148620 and Bioorganic & Medicinal Chemistry 21 (2013) 1717-1723 can be appropriately used.

Specifically, the cationic oligonucleotide used in the present invention may comprise 8 or more amino acid residues having an amino group or a guanidino group.

More specifically, the cationic oligopeptide which can be used in the present invention may be an oligopeptide consisting of 8 to 40 amino acids, comprising a cationic oligopeptide moiety comprising at least two contiguous amino acid residues of the following formula (I), and optionally comprising another non-contiguous amino acid residue other than the contiguous amino acid residues of the following formula (I), 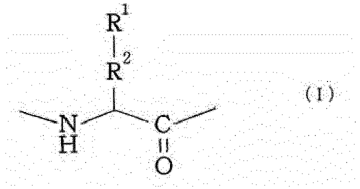 wherein R¹ is a group H₃N⁺-CH₂- or a group represented by formula (II); R² is absent or an alkylene group having 1 to 3 carbon atoms when R¹ is the group H₃N⁺-CH₂-, or R² is an alkylene group having 1 to 4 carbon atoms when R¹ is a group represented by formula (II); and in one cationic oligopeptide, all R¹ are the same and all R² are the same, and 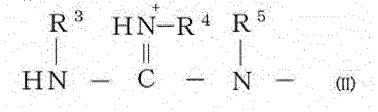 wherein R³, R⁴, and R⁵ are the same or different and are each a hydrogen atom or a methyl group.

The "amino acid residues of formula (I)" are not limited, but specific examples thereof include L-2,3-diaminopropionic acid (Dap), L-2,4-diaminobutyric acid (Dab), L-ornithine (Orn), L-lysine (Lys), L-2-amino-3-guanidinopropionic acid (Agp), L-2-amino-4-guanidinobutyric acid (Agb), or L-arginine (Arg).

The "another amino acid residue" is not specifically limited and can be appropriately selected. Examples thereof include, in addition to glycine, L-alanine, and L-proline, L-aminoproline and L-guanidinoproline as amino acids having a proline skeleton, but are not limited thereto.

In one aspect, the cationic oligopeptide moiety can be a heteromultimer comprising the "another amino acid residue." In another aspect, the cationic oligopeptide moiety can be a homomultimer without the "another amino acid residue." In consideration of the simplicity of synthesis of the cationic oligopeptide and stabilizing effects on siRNA and shRNA confirmed by the present inventors, it is preferable that the cationic oligopeptide moiety be a homomultimer composed of single type amino acids.

In this case, the amino acid as a monomer may be a natural amino acid or an unnatural amino acid. In addition, the amino acid may be L-type or D-type, and both may be intermixed in the oligopeptide molecule.

Therefore, the cationic oligopeptide may have a homomultimer of, for example, L-2,3-diaminopropionic acid (Dap), L-2,4-diaminobutyric acid (Dab), L-ornithine (Orn), L-lysine (Lys), L-2-amino-3-guanidinopropionic acid (Agp), L-2-amino-4-guanidinobutyric acid (Agb), or L-arginine (Arg) as a partial structure.

The cationic oligopeptide may be in salt form, and examples of salts that can be suitably used include a hydrochloride, an acetate, and a trifluoroacetate, but are not particularly limited thereto.

There are two types of double-stranded nucleic acids with different helical structures: type A and type B. DNA/DNA duplex has type B double-helix structure with a main groove width of 13 to 18 Å, while in RNA-RNA duplex and DNA-RNA strand have type-A double-helix structure with main groove width of 7 to 14 Å and 8 to 15 Å, respectively. Since the present invention is intended to improve the stability of siRNA, it is necessary to use a cationic oligopeptide that can bind to an RNA-RNA strand having type A double-helix structure.

The number of amino acid residues constituting the cationic oligopeptide can be 8 to 12 to improve the stability of siRNA generally consisting of 21 to 23 bases, and shRNA that yields such siRNA.

As a specific aspect actually investigated in the following Examples and produced preferred results, the cationic oligopeptide moiety can be an octamer of diaminobutyric acid having the following structure.

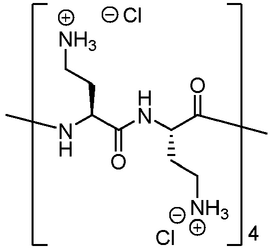

### <Folic Acid-Cationic Oligopeptide Complex>

The binding between folic acid and the cationic oligopeptide is preferably a covalent bond. The covalent bond can be binding to the N-terminus, the C-terminus, or a side chain of the cationic oligopeptide directly or via a linker.

As the linker, one usually used in the art for producing a conjugate can be appropriately used, and although not particularly limited, it can be a peptide linker consisting of amino acids such as glycine and serine. For example, the peptide linker can be a peptide consisting of 1 to 4 glycine residues. Examples of linkers actually investigated and produced preferred results in the following Examples include a linker consisting of 3 glycine residues.

In the folic acid-cationic oligopeptide complex, the ratio of folic acid to the cationic oligopeptide can be 1:1 in consideration of interaction with folate receptors and with the double-stranded nucleic acids, but is not particularly limited.

As described above, since there are two carboxyl groups in the folic acid molecule, the following two isomers can be generated when Dab8 having the structure above is attached via a reaction to the carboxyl groups of folic acid. In the present specification, these compounds are denoted Fol-Dab8A and Fol-Dab8B for convenience.

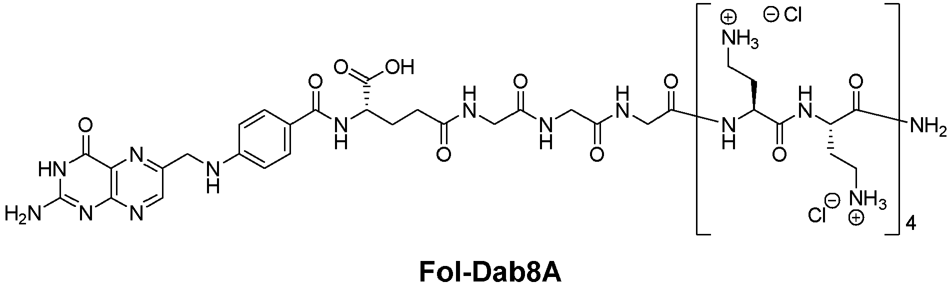

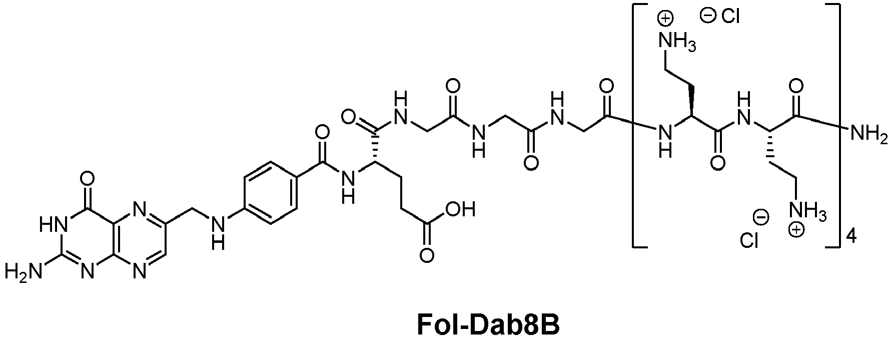

In Fol-Dab8A and Fol-Dab8B above, three glycines are used as a linker, but the length of the linker can be appropriately changed.

Depending on the type of cationic oligopeptide used to form the complex, the folic acid-cationic oligopeptide complex may be obtained in salt form. Examples of salts that can be suitably used include a hydrochloride, an acetate, and a trifluoroacetate, but are not particularly limited thereto.

The folic acid-cationic oligopeptide complex enhances the stability of siRNA and shRNA and enables targeted delivery to cells having a folate receptor.

Note that in the Examples, in order to demonstrate the effectiveness of Fol-Dab8A and Fol-Dab8B, the cationic oligopeptide Dab8 having no folic acid but having tyrosine (with N-terminus protected by an acetyl group) and three glycines on the N-terminus to enable UV detection and quantification is used as a control compound, which is referred to as Dab8 for convenience in Examples.

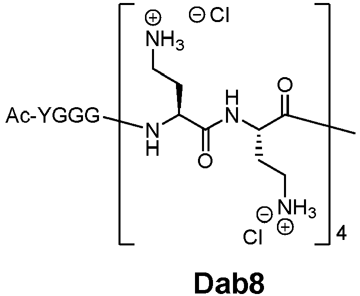

### [Ac-YGGG indicates N-acetyl-L-tyrosine-glycine-glycine-glycine.]

The nucleic acid delivery enhancer of the present invention can enhance the delivery of siRNA or shRNA to cells *in vitro* and *in vivo,* as demonstrated in Examples. The nucleic acid delivery enhancer of the present invention can specifically enhance the delivery to cells that highly express folate receptors, especially cancer cells.

### <Anti-tumor Agent>

The present invention also provides a nucleic acid formulation comprising: siRNA or shRNA capable of binding to mRNA or snoRNA expressed in pancreatic cancer cells to inhibit its expression, and the delivery enhancer of the present invention. The nucleic acid formulation of the present invention can effectively suppress tumor growth, invasion, and metastasis of pancreatic cancer. Therefore, the nucleic acid formulation of the present invention acts as a tumor growth inhibitor, an invasion and metastasis inhibitor, and an anti-tumor agent for pancreatic cancer.

In the present invention, the siRNA or shRNA and the folic acid-cationic oligopeptide complex can be bound via a further covalent bond, or the interaction without a covalent bond provided by the negative charge of the siRNA or shRNA and the positive charge of the cationic oligopeptide, can allow the folic acid-cationic oligopeptide complex to partially enter the main groove of the siRNA or shRNA to form a stable structure. However, the invention is not intended to be bound by the mechanism of this stabilizing effect.

### <RNAi Molecule>

It is known that siRNA and shRNA target a specific mRNA and block its translation (expression) by a mechanism called RNA interference. The number of bases of the target sequence is not particularly limited and can be selected in the range of 15 to 500 bases. The siRNA is a short double-stranded RNA molecule, and the shRNA is a hairpin RNA that can be processed by Dicer *in vivo* to generate siRNA. Herein, siRNA and shRNA may be included and described as "RNAi molecules."

The siRNA is a double-stranded RNA in which a sense strand homologous to a partial nucleotide sequence of the target RNA and an antisense strand that can hybridize with the sense strand hybridize, and its 3' end is usually OH while its 5' end is phosphorylated, and the 3' end side may protrude by 1 base or more and 4 bases or less.

In contrast, the shRNA, which consists of single-stranded RNA, has a sense strand homologous to a partial nucleotide sequence of the target mRNA and an antisense strand that can hybridize with the sense strand, connected by a linker region, and thus has a hairpin-like structure as a whole. The shRNA may have a 3' end protruding by 1 base or more and 4 bases or less, and the 3' protruding end may be composed of DNA. The shRNA is degraded intracellularly as described above, causing RNA interference in the same manner as the siRNA. Therefore, shRNA may be used instead of siRNA.

The siRNA or shRNA used for the purpose of the present invention is siRNA or shRNA capable of binding to mRNA or snoRNA expressed in pancreatic cancer cells to inhibit its expression.

The "mRNA or snoRNA expressed in pancreatic cancer cells" described herein is not particularly limited but may bind to insulin-like growth factor 2 mRNA-binding protein 3 (IGF2BP3).

As described above, human IGF2BP3 is present in cell membrane protrusions in pancreatic cancer cells, and various mRNAs bind to the IGF2BP3 and accumulate in the cell membrane protrusions. Inhibition of the mRNAs binding to IGF2BP3 in these cell membrane protrusions by RNA interference can effectively suppress tumor growth, invasion, and metastasis in pancreatic cancer.

Examples of the mRNAs expressed in pancreatic cancer cells for which anti-tumor effect on pancreatic cancer utilizing RNA interference has been confirmed by the present inventors include mRNAs of NUP85, WASF2, ARHGEF4, CCDC88A, LAMTOR2, and mTOR.

### <NUP85>

NUP85 (nucleoporin 85) is a protein belonging to the nucleoporin protein family, a component of a nuclear pore complex that forms entry/exit points regulating the transfer of macromolecules between the cell nucleus and cytoplasm. Information such as amino acid sequence of human NUP85 and nucleotide sequence of mRNA encoding the same is listed in a database such as NCBI as Gene ID: 79902, NCBI reference sequence: NM_024844, and the like.

### <WASF2>

Wiskott-Aldrich syndrome, one type of primary immunodeficiency, is a disease characterized by thrombocytopenia with size reduction, eczema, and susceptibility to infection. Wiskott-Aldrich syndrome protein family member 2 (WASF2) belongs to the Wiskott-Aldrich syndrome protein family, which forms a multi-protein complex connecting a receptor kinase with actin. Information such as amino acid sequence of human WASF2 and nucleotide sequence of mRNA encoding the same is listed in a database such as NCBI as Gene ID: 10163, NCBI reference sequence: NM_006990, and the like.

### <ARHGEF4>

ARHGEF4 (Rho guanine nucleotide exchange factor 4) is a protein involved in intracellular processes initiated by stimuli that function via G protein-coupled states. Information such as amino acid sequence of human ARHGEF4 and nucleotide sequence of mRNA encoding the same is listed in a database such as NCBI as Gene ID: 50649, NCBI reference sequence: NM_015320, and the like.

### <CCDC88A>

CCDC88A (coiled-coil domain containing protein 88A) is a gene encoding Girdin protein, an actin-binding protein. Information such as nucleotide sequence of human CCDC88A mRNA is listed in a database such as NCBI as Gene ID: 55704, NCBI reference sequence: NM_001135597, and the like.

### <LAMTOR2>

LAMTOR2 (late endosomal/lysosomal adapter, MAPK and mTOR activator 2) is a regulator of Langerhans cell homeostasis and has been reported to be involved in signal transduction and mTOR cascades. Information such as amino acid sequence of human LAMTOR2 and nucleotide sequence of mRNA encoding the same is listed in a database such as NCBI as Gene ID: 28956, NCBI reference sequence: NM_014017, and the like.

### <mTOR>

mTOR (mammalian target of rapamycin kinase) is one type of protein kinases involved in intracellular signal transduction in mammals and the like. Information such as amino acid sequence of human mTOR and nucleotide sequence of mRNA encoding the same is listed in a database such as NCBI as Gene ID: 2475, NCBI reference sequence: NM_004958, and the like.

As well known in the art, mRNA is RNA that is transcribed from a gene (DNA) and contains information encoding a protein. siRNA is usually double-stranded, and it is known that one strand (antisense strand) forms a complex called RISC (RNA-induced silencing complex) with a specific protein after the double strand is dissociated. RISC recognizes and binds to mRNA having a sequence homologous to the nucleotide sequence of the sense strand of siRNA, and cleaves the mRNA by RNase III-like enzymatic activity. On the other hand, shRNA can give rise to siRNA after processing in delivered cells and then function in the same manner.

In contrast, snoRNA is a non-coding RNA (small nuclear RNA) that exists in the nucleolus and has been reported to be a group of RNA molecules having functions such as guiding chemical modifications of ribosomal RNA and other RNA methylation and pseudouridylation (for example, see, Mol. Biol. Cell, 2004, 15: 281-293; J. Biol. Chem, 2015, 290: 11741-11748).

The present inventors have recently found that SNORA18 and SNORA22, which belong to such snoRNAs, have an ability to bind to IGF2BP3 and are involved in the movement or invasion of pancreatic cancer cells. It has also been found that snoRNAs bind to KH-type splicing regulatory protein (KHSRP) and are localized in the cytoplasmic P-body (Oncotarget, 2020, Vol. 11, No. 2, pp. 131-147).

The present inventors have found that siRNA or shRNA for these snoRNAs can also recognize and bind to snoRNA and knock down the snoRNA, thereby suppressing tumor growth, invasion, and metastasis of pancreatic cancer.

### <SNORA18>

The small nucleolus RNA SNORA18 has been reported as a member of RNAs that guide modification sites from uridine to pseudouridine. Information such as nucleotide sequence of human SNORA18 is listed in a database such as NCBI as Gene ID: 677805, NCBI reference sequence: NR_002959, and the like.

### <SNORA22>

The small nucleolus RNA SNORA22 has also been reported as a member of RNAs that guide modification sites from uridine to pseudouridine. Information such as nucleotide sequence of human SNORA22 is listed in a database such as NCBI as Gene ID: 677807, NCBI reference sequence: NR_002961, and the like.

Therefore, examples of the mRNA or snoRNA expressed in pancreatic cancer cells that can be targeted by siRNA or shRNA in the present invention include, but are not particularly limited to, those selected from the group consisting of SNORA18 snoRNA, NUP85 mRNA, WASF2 mRNA, and SNORA22 snoRNA.

When targeting mRNA, the target mRNA that siRNA or shRNA can hybridize may include 3' UTR, 5' UTR, an exon, an intron, a coding region, a translation initiation region, a translation termination region, or another nucleic acid region.

The siRNA and shRNA used in the present invention can knock down mRNA or snoRNA to inhibit its function. Therefore, more specifically, the siRNA and shRNA of the present invention consist of a nucleotide sequence substantially complementary to a specific nucleotide sequence of the target RNA. However, the siRNA and shRNA may have one or two mismatches to the target nucleotide sequences. If the nucleotide sequence of the mRNA or snoRNA to be targeted has been obtained, those skilled in the art can design and synthesize appropriate siRNA and shRNA with high specificity by selecting regions that are not homologous to nucleotide sequences of nucleic acids other than the target sequence and that are suitable for inhibition of the expression of the target sequence and knockdown thereof.

For example, siRNA is known to have a high RNA interference effect if the following conditions are met:
(1) an antisense strand has A or U at the 5' end;
(2) a sense strand has G or C at the 5' end; and
(3) 4 or more of 7 bases at the 5' end of the antisense strand are A or U.
Therefore, the siRNA used in the present invention can be one having such nucleotide sequence but is not limited thereto. Similarly, the shRNA used in the present invention can give rise to such siRNA after intracellular processing but is not limited thereto.

Determination of effective siRNA and shRNA sequences for a certain target sequence can also be performed using a program available via the Internet.

Inhibition/suppression of expression or knockdown herein refer to degradation of target mRNA or snoRNA, inhibition and suppression of translation into an encoded protein. Suppression includes reducing the amount of target mRNA/snoRNA in a cell or a cell group (pancreatic cancer tissue) by 20% or more, 30% or more, 40% or more, 50% or more, 60% or more, 70% or more, 80% or more, or 90% or more as compared with the control. The inhibition and suppression of expression or knockdown may be determined by any method known in the art, for example, by a method based on RT-PCR.

The siRNA and shRNA may be composed of only natural (unmodified) nucleotides, but some or all of the nucleotides may be modified. The chemical stability of the siRNA and shRNA can be increased by using any modifications known in the art. However, it is not desirable that the modifications reduce intended activities of the siRNA and shRNA. For example, it will be understood that no modifications are used that interfere with the intracellular processing of the shRNA. Modifications as unnatural nucleotides can be sugar and/or base modifications.

Examples of the sugar modifications include, but are not limited to, a bicyclic sugar, and 5'-vinyl, 5'-methyl, 4'-S, 2'-F, 2'-OCH₃ (2'-OMe), and 2'-O(CH₂)₂OCH₃ substituents. The bicyclic sugar is generally referred to as a bridged nucleic acid (BNA), and examples thereof include, but are not limited to, LNA (Locked Nucleic Acid^{®}), 2,4-BNA, and ENA (ethyleneoxy(4-(CH₂)₂-O-2)BNA). Alternatively, the sugar may also contain deoxyribose in part instead of ribose, and in this case, DNA and RNA may be intermixed in one or both oligonucleotide strands of siRNA or in the oligonucleotide strand of shRNA. The siRNA is preferably an RNA-RNA duplex. The shRNA is preferably an RNA strand. As the sugar modification, for example, 2'-OMe modification can be preferably used.

Examples of base modifications include, but are not limited to,
5-methylation, 5-fluorination, 5-bromination, 5-iodination, and N4-methylation of cytosine;
N6-methylation and 8-bromination of adenine;
N2-methylation and 8-bromination of guanine; and
5-fluorination, 5-bromination, 5-iodination, and 5-hydroxylation of uracil.

The same or different nucleotides in siRNA and shRNA can have sugar and base modifications as described above.

The siRNA and shRNA can also contain altered internucleoside bonds.

The altered internucleoside bonds can be, for example, phosphorothioate, phosphorodithioate, boranophosphate, phosphorodiamidate, and phosphoramidate bonds, which may be present instead of naturally-occurring phosphodiester bonds.

At least one of the internucleoside bonds of the siRNA and shRNA can be an altered internucleoside bond. Alternatively, at least two, three, four, or more of the internucleoside bonds of the siRNA and shRNA can be altered internucleoside bonds. The altered internucleoside bonds are preferably phosphorothioate bonds.

In siRNA and shRNA, different nucleotides in the same strand can independently undergo different modifications. The same nucleotide can have a modified internucleoside bond (for example, a phosphorothioate bond) and further have a modified sugar (for example, bicyclic sugar). The same nucleotide can also have a modified nucleobase (for example, 5-methylcytosine) and further have a modified sugar (for example, 2'-OMe modification and bicyclic sugar).

The siRNA and shRNA of the present invention can be manufactured by a method known in the art. For example, siRNA and shRNA can be manufactured by synthesis using a commercially available automated nucleic acid synthesizer and then purification using an ionexchange column or a reverse-phase column. Alternatively, siRNA and shRNA can be obtained by ordering from a manufacturer (for example, JEEN DESIGN Co., Ltd.) by specifying nucleotide sequences, modification sites, and types.

In addition, functional molecules such as labeled compounds (such as fluorescent proteins and luciferase), compounds for purification (such as biotin, avidin, His-tag peptide, GST-tag peptide, and FLAG-tag peptide) may be attached to siRNA and shRNA. The binding may be a direct binding or an indirect bond via another substance, but it is preferably a direct binding such as a covalent bond.

The anti-tumor agent of the present invention may comprise, but is not limited to, a folic acid-cationic oligopeptide complex in an amount of 0.5 to 10 equivalents, preferably 1 to 5 equivalents, and more preferably 1 to 3 equivalents per molecule of siRNA or shRNA.

As demonstrated in Examples, the combined use of siRNA or shRNA with a folic acid-cationic oligopeptide complex can significantly suppress tumor growth and invasion of pancreatic cancer. Since the folic acid-cationic oligopeptide complex of the present invention exerts a sufficient effect only by adding a small amount of about 1 to 3 equivalents to siRNA or shRNA, it is expected to be an extremely useful therapeutic measure for pancreatic cancer.

The anti-tumor agent of the present invention is not specifically limited as long as the active ingredient can be delivered to the target site, pancreatic cancer tissue, and may be, for example, an injection, a liquid, or a sustained-release agent. Water is preferable as the solvent for these formulations, but it is preferable to use saline, PBS, serum albumin solution, or the like so that the formulation finally becomes an isotonic solution or a substantially isotonic solution.

### <Pharmaceutical Composition>

The present invention also provides a pharmaceutical composition comprising one or more anti-tumor agents of the present invention.

The target site of the anti-tumor agent according to the present invention may be not only pancreas but also lymph nodes or other organs to which pancreatic cancer cells have metastasized. In addition, an injection is preferable as a dosage form to more reliably deliver the active ingredient to the target site.

The pharmaceutical composition can copmprise carriers, excipients, stabilizers, disintegrants, surfactants, binders, lubricants, emulsifiers, suspensions, antioxidants, odorants, fillers, solubilizers, coating agents, colorants, flavoring agents, preservatives, buffers, and other agents commonly used in the pharmaceutical filed. Specific examples thereof include water, saline, other aqueous solvents, pharmaceutically acceptable organic solvents, mannitol, lactose, starch, microcrystalline cellulose, glucose, calcium, polyvinyl alcohol, collagen, polyvinylpyrrolidone, carboxyvinyl polymers, sodium alginate, water-soluble dextran, water-soluble dextrin, sodium carboxymethyl starch, pectin, gum arabic, xanthan gum, casein, gelatin, agar, propylene glycol, polyethylene glycol, vaseline, paraffin, glycerin, stearyl alcohol, stearic acid, and sorbitol.

The pharmaceutical composition can be orally or parenterally administered to a subject. Examples of parenteral administration include, but are not limited to, subcutaneous, intravenous, intraperitoneal, intratumoral, and other injection or infusion, and administration during endoscopic or laparoscopic treatment.

The dose and frequency of administration of the pharmaceutical composition of the present invention may be appropriately adjusted according to each dosage form, and the age, sex, weight, severity of disease, and the like of the patient. For example, the pharmaceutical composition comprises siRNA or shRNA in an amount of 0.001 mg/kg/day to 1 mg/kg/day, 0.005 mg/kg/day to 0.5 mg/kg/day, or 0.01 mg/kg/day to 0.1 mg/kg/day, and the amount of the folic acid-cationic oligopeptide complex can be determined to be 0.5 to 10 equivalents relative to this amount for administration.

The pharmaceutical composition can be administered in single or multiple doses, for example, at interval of 1 day, 2 days, 3 days, 4 days, 5 days, 6 days, 1 week, 2 weeks, 3 weeks, or 1 month.

The subject to whom the pharmaceutical composition is administered can be mammals such as primates such as humans and monkeys, and non-primates such as cattle, pigs, sheep, horses, cats, dogs, guinea pigs, rats, and mice, but are more preferably humans. In addition, the subject may also be, for example, pancreatic cancer model animals transplanted with human pancreatic cancer cells, which can be used for evaluation of efficacy in humans.

The pancreatic cancer model animals are not particularly limited, and for example, non-human animals transplanted with the cancer organoid described in JP Patent Application Publication No. 2018-110575 A1 can be used. The "organoid" is an organ three-dimensionally produced *in vitro* and refers to an aggregate of cells which are specific to a certain organ. Organoids of various organs have already been produced in the art. The "cancer organoid" described in JP Patent Application Publication No. 2018-110575 A1 is an organoid that reproduces the microenvironment of cancer tissue. The present inventors' group has modified the approach of JP Patent Application Publication No. 2018-110575 A1 to produce a mouse model of human pancreatic cancer useful for determining the effect of pancreatic cancer therapeutic agents (JP Patent Application No. 2020-078771), and this mouse model can also be suitably used for evaluating the efficacy of the anti-tumor agent and the pharmaceutical composition of the present invention in humans.

### <Combined Formulation>

The present invention also provides a combined formulation for suppressing tumor growth, invasion, and metastasis of pancreatic cancer, comprising siRNA or shRNA capable of binding to mRNA or snoRNA expressed in pancreatic cancer cells to inhibit its expression and the folic acid-cationic oligopeptide complex.

As can be recognized from the descriptions in the present specification, the siRNA or shRNA and the folic acid-cationic oligopeptide complex are not covalently bound and, without wishing to be limited to any mechanism, it is intended that the folic acid-cationic oligopeptide complex is partially inserted into the main groove of the siRNA or shRNA to stabilize the siRNA or shRNA.

Therefore, the siRNA or shRNA and the folic acid-cationic oligopeptide complex may not be contained in the same composition in advance. In other words, a formulation comprising siRNA or shRNA and a formulation comprising the folic acid-cationic oligopeptide complex may be prepared separately and combined before administration.

Alternatively, the two formulations above can be administered separately. However, in consideration of the stabilization of siRNA or shRNA by the folic acid-cationic oligopeptide complex, these two formulations are preferably administered simultaneously (or consecutively) and by the same route of administration, rather than at different times or by different routes of administration.

### <Kit>

The present invention also provides a pharmaceutical kit for treating pancreatic cancer, comprising siRNA or shRNA capable of binding to mRNA or snoRNA expressed in pancreatic cancer cells to inhibit its expression and the folic acid-cationic oligopeptide complex. The kit may include siRNA or shRNA and the folic acid-cationic oligopeptide complex, as well as other drugs that can be, or are suitably administered simultaneously, carriers, instructions for administration, and the like.

### Examples

The present invention is described in more detail by the following Examples, but the present invention is not limited by these Examples.

### [Example 1: Synthesis of Folic Acid-Cationic Oligopeptide Complex]

A folic acid-cationic oligopeptide complex was synthesized as shown below. Note that the reagents and analyzers used in this Example are as follows.

### <Reagent>

Fmoc amino acid derivatives and resin as a carrier for peptide solid-phase synthesis were purchased from WATANABE CHEMICAL INDUSTRIES, LTD., folic acid was purchased from Tokyo Chemical Industry Co., Ltd., and each was used as-is. Each peptide chain was synthesized by Fmoc solid-phase synthesis method using Fmoc-NH-SAL-PEG resin as a solid-phase carrier. As the Fmoc-AA-OH reagent, Fmoc-Dab(Boc)-OH, Fmoc-Gly-OH, and Fmoc-Tyr(t-Bu)-OH were used.

### <ESI MS>

Varian 910-MS (JASCO Corporation)

### <UV-visible spectrophotometer>

V-550 (JASCO Corporation)

### <Temperature variable UV-Vis spectrophotometer>

UV-1650PC (Shimadzu Corporation)

### <Spectrofluorometer>

FP-6500 (JASCO Corporation)

### <HPLC>

Pump: PU-2080i plus (JASCO Corporation)
Detector: UV-2075i plus (JASCO Corporation)
Low-pressure gradient unit: LG-2080-02 (JASCO Corporation)
Degasser: DG-2080-53 (JASCO Corporation)
Reverse-phase column: µ-Bondasphere 150 × 3.9 mm C18, 5 µm, 100 Å (Waters Corporation); SunFire C18 OBD, 5 µm, 19 × 150 mm (Waters Corporation)

### <Step 1: Coupling Procedure>

First, an octamer of L-2,4-diaminobutyric acid (Dab8) was synthesized by Fmoc solid-phase synthesis. The solid-phase carrier was added to PetiSyzer (HiPep Laboratories) so that introduced amino groups reached 13 µmol, washed five times with 1.3 mL of dimethylformamide (DMF), and then 1.3 mL of DMF was added thereto and allowed to stand for 1 hour or longer to swell the carrier.

(i) After swelling, the carrier was washed 5 times with 1.3 mL of DMF, then 1.3 mL of 25% piperidine/DMF solution was added thereto and reacted for 5 minutes to remove the Fmoc group. At that time, the mixture was stirred several times with a vortex mixer.
(ii) Subsequently, the resultant was washed five times with 1.3 mL of DMF, and then 5 equivalents of Fmoc-amino acid (Fmoc-AA-OH) relative to the amino group on the resin, N-[(1H-benzotriazol-1-yl)(dimethylamino)methylene] -N-methylmethan aminiumhexafluorophosphate-N-oxide (HATU·H₂O, 5 equivalents) as a condensing reagent, diisopropylethylamine (DIPEA, 10 equivalents), and DMF as a reaction solvent were added thereto so that the total volume reached 1.3 mL and subjected to condensation reaction for 15 minutes. At that time, the mixture was stirred several times with a vortex mixer.

After repeating procedures (i) and (ii) up to the N-terminal amino acid, the Fmoc group was removed by performing the procedure (i) again to synthesize NH₂-GGG-Dab8 on the solid-phase carrier. Subsequently, the resultant was washed five times with 1.3 mL of DMF, 2 equivalents of folic acid, and 2 equivalents of N,N'-dicyclohexylcarbodiimide (DCC) relative to the amino group on the resin dissolved in 1.5 mL of a DMF-dimethylsulfoxide (DMSO) mixed solvent (1:1, v/v) and allowed to stand for 6 hours were added in an amount of 1.3 mL and subjected to condensation reaction for 14 hours.

### <Step 2: Deprotection, Resin Removal, and Purification>

The resin was washed five times each with DMF and CHCl₃ and dried under reduced pressure in a desiccator. The resulting resin was stirred in trifluoroacetic acid (TFA)-triisopyropylsilane-H₂O mixed solvent (96.5/1.0/2.5, v/v/v) at room temperature for 1.5 hours to perform deprotection and resin removal. The resin was removed by filtration, the solvent was vaporized under argon gas flow, and then Et₂O was added to precipitate the peptides. After repeated centrifugation procedures to remove the supernatant three times, the Et₂O was vaporized under argon gas flow to obtain a crude product (folic acid-cationic oligopeptide).

The thus obtained crude product was dissolved in 1 mL of OTSUKA DISTILLED WATER (manufactured by Otsuka Pharmaceutical Factory, Inc.) and then purified by reverse-phase HPLC with the following conditions.

Gradient cycle: Ratio of solvent B (0.05% TFA / CH₃CN) in solvent A (0.05% TFA / H₂O), 0% to 5% in a 5-minute linear gradient; 5% to 25% in 40 minutes; and 25% to 100% in 5 minutes
Measured temperature: 30°C
Flow rate: 0.5 mL/min

Figure 1 shows HPLC analysis results of the Fol-Dab8 crude product. Because folic acid contains two carboxy groups, two separate peaks were eluted for Dab8 having folic acid introduced thereto (described as Fol-Dab8). In the HPLC analysis, the one eluted first was designated as Fol-Dab8A, and the one eluted thereafter was designated as Fol-Dab8B. Each chemical structural formula is shown below.

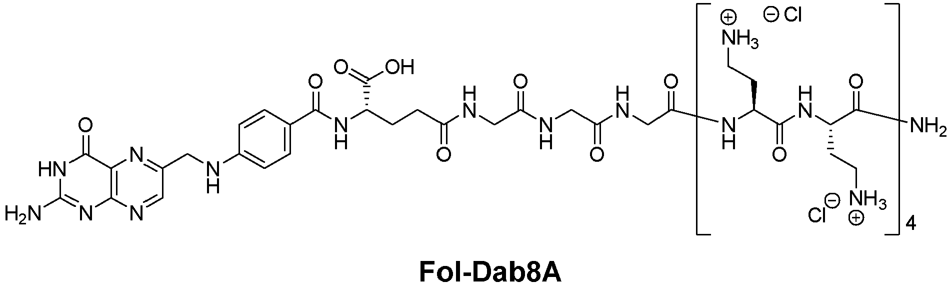

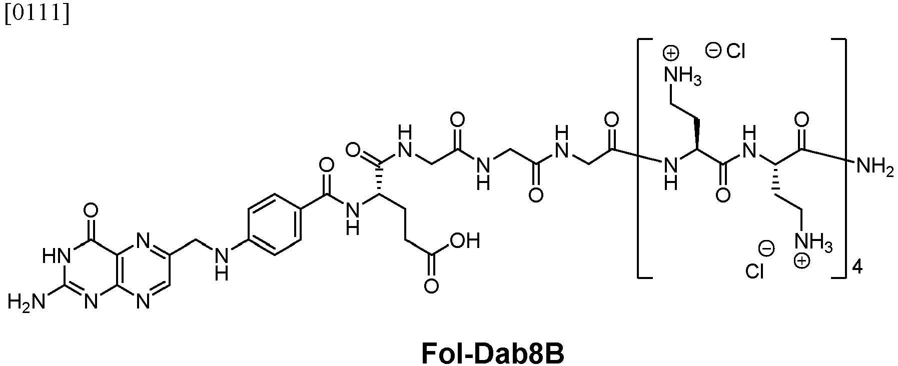

The solution separated by HPLC was lyophilized to obtain a yellow powder. Purified Fol-Dab8A or Fol-Dab8B was dissolved in OTSUKA DISTILLED WATER, and the molar absorption coefficient of folic acid was calculated in advance (molar absorption coefficient at 368 nm wavelength: 7,967 L/mol·cm), and the yield was calculated from UV absorption of folic acid. Dab8 was obtained as a white powder by synthesis of NH₂-GGG-Dab8 on the solid-phase carrier followed by coupling procedure with tyrosine whose N-terminus was protected by an acetyl group, and purification by HPLC after <Step 2> procedure, and the yield was calculated from the UV absorption of Tyr. Each peptide was identified by mass spectrometry (ESI-MS) (Dab8 [M+H]⁺m/z calculated: 1,194.681, found: 1,194.682; Fol-Dab8A [M+H]⁺ m/z calculated: 1,412.737, found: 1,412.738; Fol-Dab8B [M+H]⁺m/z calculated: 1,412.737, found: 1,412.739).

### [Example 2: Melting Temperature (Tm) Analysis]

The melting temperature of the nucleic acid duplex was measured in the presence and absence of the cationic oligopeptide or folic acid-cationic oligopeptide complex synthesized in Example 1.

In this Example, the cationic oligopeptide (Dab8) or the folic acid-cationic oligopeptide (Fol-Dab8A or Fol-Dab8B) was added to an annealed RNA duplex, and the melting temperature (Tm value) was measured. As the RNA duplex, an oligonucleotide pair consisting of the following sequences was used as an siRNA.
5'-r(GUCAUCACACUGAAUACCA)dTdT-3' (SEQ ID NO: 1)
5'-r(UGGUAUUCAGUGUGAUGAC)dTdT-3' (SEQ ID NO: 2)

After 144 µL of 50 µM nucleic acid solution was prepared and kept at 95°C for 5 minutes, it was slowly cooled to 4°C at -0.5°C/min. The solution was added to a mixed solution of pH 7.0 buffer containing 200 mM NaCl and 20 mM Na₂HPO₄-NaH₂PO₄ in OTSUKA DISTILLED WATER, and 0.1 mM peptide aqueous solution, and samples were adjusted to a final concentration of 10 mM Na₂HPO₄-NaH₂PO₄, 100 mM NaCl, 4 µM nucleic acid duplex, and 0, 4, 8, 12, 16, and 20 µM peptide (0, 1, 2, 3, 4, and 5 equivalents, respectively).

The temperature was increased from 20°C to 95°C at 0.5°C/min, and the absorbance at 260 nm was measured to determine a melting curve. The absorbance at 320 nm was measured to remove background noise, and a melting curve was created by subtracting the absorbance at 320 nm from the absorbance at 260 nm, and the Tm value was determined by a median method.

As a result, as shown in Table 1, when no cationic oligopeptide was added to the RNA duplex, the Tm value was 72.3°C. In contrast, the Tm value increased when one or more equivalents of Dab8, Fol-Dab8A, or Fol-Dab8B were added. In other words, it was suggested that both Fol-Dab8A and B, like Dab8, bind to RNA duplexes and improve their thermodynamic stability.

**[Table 1]**

| | Cationic peptide | | *Tm* | Δ*Tm* |
|---|---|---|---|---|
| 1 | None | | 72.3 | - |
| 2 | Dab8 | 1 equivalent | 77.2 | 4.9 |
| 3 | | 2 equivalents | 79.1 | 6.8 |
| 4 | | 3 equivalents | 79.9 | 7.6 |
| 5 | | 4 equivalents | 80.2 | 7.9 |
| 6 | | 5 equivalents | 80.6 | 8.3 |
| 7 | Fol-Dab8A | 1 equivalent | 75.2 | 2.9 |
| 8 | | 2 equivalents | 76.8 | 4.5 |
| 9 | | 3 equivalents | 77.7 | 5.4 |
| 10 | | 4 equivalents | 78.7 | 6.4 |
| 11 | | 5 equivalents | 79.1 | 6.8 |
| 12 | Fol-Dab8B | 1 equivalent | 74.5 | 2.2 |
| 13 | | 2 equivalents | 76.4 | 4.1 |
| 14 | | 3 equivalents | 77.7 | 5.4 |
| 15 | | 4 equivalents | 78.5 | 6.2 |
| 16 | | 5 equivalents | 79.8 | 7.5 |

### [Example 3: Evaluation of RNase A Resistance]

RNA-degrading enzyme resistance of the nucleic acid duplex was tested in the presence of a cationic oligopeptide.

In this Example, peptides were added to the annealed RNA duplex to form a complex, and the rate of RNA degradation was measured.

After adding OTSUKA DISTILLED WATER, 100 mM Tris-HCl, 1 M NaCl buffer (pH 7.3) to a PCR tube, 0.1 mM of 6-FAM modified single-stranded RNA aqueous solution with a fluorescent group at the 5' end (5'-FAM-r(GUCAUCACACUGAAUACCA)dTdT-3', SEQ ID NO: 1) and 0.1 mM of Dabcyl-modified single-stranded RNA aqueous solution with a quenching group at the 3' end (5'-r(UGGUAUUCAGUGUGAUGAC)dTdT-dabcyl-3', SEQ ID NO: 2) were mixed in equal volume to prepare the final concentration of 10 mM Tris-HCl, 100 mM NaCl, and 10 µM siRNA. Subsequently, the mixture was kept at 95°C for 5 minutes and then slowly cooled to 4°C at -0.5°C/min.

To a quartz cell, 10 mM Tris-HCl, 100 mM NaCl, 10 µM siRNA aqueous solution, and 0.1 mM cationic oligopeptide aqueous solution were added, and prepared in 3 mL so that the final concentration reached 10 mM Tris-HCl, 100 mM NaCl, 10 nM siRNA, and 0, 10, 20, and 30 nM peptide (0, 1, 2, and 3 equivalents, respectively). Thereafter, 15 µL of 100 µg/mL bovine pancreas-derived RNase A (manufactured by F. Hoffmann-La Roche, Ltd.) was added with stirring at 37°C, and fluorescence intensity measurement was started to track changes over time (excitation wavelength: 490 nm; measurement wavelength: 520 nm; and measurement time: 60 minutes).

Results are shown in Figure 2. As the RNA duplex dissociates, the distance between the fluorescent and quenching groups becomes longer, and the fluorescence intensity increases. The rate of increase in fluorescence intensity becomes slower when 1 or more equivalents of Dab8, Fol-Dab8A, or Fol-Dab8B are added, suggesting that, as with Dab8, the degradation enzyme resistance of the RNA duplex is improved by Fol-Dab8A and B.

### [Example 4: Confirmation of Uptake of siRNA into Pancreatic Cancer Cells]

Since it is difficult to distinguish between intracellularly incorporated labels and non-incorporated labels by flow cytometry, the uptake of siRNA into pancreatic cancer cells was observed using a confocal microscope.

The pancreatic cancer cell line S2-013 (Cell Resource Center for Biomedical Research, Institute of Development, Aging and Cancer, Tohoku University, ID: TKG 0709) was seeded in a 4-well chamber slide (Thermo Fisher Scientific Inc.) at a cell density of 2 × 10⁴ cells/well, and scrambled control siRNA (SEQ ID NOs: 3 and 4) labeled with Alexa647 (Thermo Fisher Scientific Inc.), and Dab8, Fol-Dab8A, or Fol-Dab8B in 1 to 3 equivalents to the siRNA were added and then cultured for 48 hours. The concentration of siRNA used was 8.28 µg/mL in 25 µL, and Dab8, Fol-Dab8A, and Fol-Dab8B were diluted 25-fold from the stock solution (Dab8: 119.3 µg/mL, Fol-Dab8A and Fol-Dab8B: 141.2 µg/mL) and used. The amounts corresponding to 1, 2, and 3 equivalents relative to siRNA are 3.9 µL, 7.8 µL, and 11.7 µL, respectively.

The next day, the cells were fixed with 4% paraformaldehyde, sealed with a DAPI-containing encapsulant, and then observed with an all-in-one fluorescence microscope (BZ-X800, Keyence Corporation). As a result, as shown in FIG. 3A, the uptake of siRNA into cells was confirmed by the fluorescence of Alexa647. On the other hand, when only siRNA was added to the culture solution of the pancreatic cancer cell line S2-013 and cultured, it was confirmed by the fluorescence of Alexa647 that almost no siRNA was taken up by the cells (FIG. 3B).

To quantify the uptake, the hybrid cell count function of the Keyence analysis software BZ-X800 Analyzer was used to count cells into which siRNA was taken up in the presence of Dab8, Fol-Dab8A, and Fol-Dab8B. Determination was made by measuring cells with dark staining of at least 1/3 of the cell nuclei and cells with dark clumps in the periphery of the cell nuclei.

As a result, as shown in Figure 3C, when the cationic oligopeptide (Dab8) and the folic acid-cationic oligopeptide complexes (Fol-Dab8A and Fol-Dab8B) were used, 20 to 40% uptake of the added siRNA into cells was observed. In addition, the highest incorporation efficiency was observed in 3 equivalents for Fol-Dab8A and 1 equivalent for Fol-Dab8B, and the incorporation efficiency was higher than Dab8 of 1 to 3 equivalents added.

### [Example 5: Knockdown Effect of siRNA with addition of Folic Acid-Cationic Peptide]

The knockdown effects of siRNA on SNORA18, NUP85, WASF2, and SNORA22 in the presence of the cationic oligopeptide or the folic acid-cationic oligopeptide complex were each investigated.

Table 2 shows the siRNA sequences used in this Example. As is usually done in the art, each siRNA used was a double-stranded product formed by a sense strand and an antisense strand as shown in Table 2.

**[Table 2]**

| | Sense strand | SEQ ID NO | Antisense strand | SEQ ID NO |
|---|---|---|---|---|
| Scrambled control | 5'- UUCUCCGAACGUGUCACGUAU | 3 | 5'- AUACGUGACACGUUCGGAGAA | 4 |
| SNORA18 | 5'- UUUACUUUACUCACAGGACUA | 5 | 5'- UAGUCCUGUGAGUAAAGUAAA | 6 |
| NUP85 | 5'- CAGCGGCAGAUGACUGAACAA | 7 | 5'- UUGUUCAGUCAUCUGCCGCUG | 8 |
| WASF2 | 5'- UAGGAUUAGAUCAUUAGCUCA | 9 | 5'- UGAGCUAAUGAUCUAAUCCUA | 10 |
| SNORA22 | 5'- CUUGGCUUUGACCCUGUGCUA | 11 | 5'- UAGCACAGGGUCAAAGCCAAG | 12 |

Scrambled control siRNA (SEQ ID NOs: 3 and 4), SNORA18 siRNA (SEQ ID NOs: 5 and 6), NUP85 siRNA (SEQ ID NOs: 7 and 8), WASF2 siRNA (SEQ ID NOs: 9 and 10), and SNORA22 siRNA (SEQ ID NOs: 11 and 12), to which a cationic oligopeptide or a folic acid-cationic peptide was added (a complex of Dab8 with siRNA in 1 equivalent, a complex of Fol-Dab8A with siRNA in 3 equivalents, and a complex of Fol-Dab8B with siRNA in 1 equivalent), were each added to the culture solution the S2-013 cells in a 6-well plate (Thermo Fisher Scientific Inc.) (1.0 × 10⁵ cells/well), and the cells were recovered after 48 hours.

Semi-quantitative RT-PCR was performed using RNA from the recovered cells to confirm the knockdown effects of SNORA18, NUP85, WASF2, and SNORA22 in the cells.

Specifically, the total RNA obtained from the S2-013 cells was reverse-transcribed using StrataScript reverse transcriptase (Agilent) and random primers. Appropriate dilutions of each single-stranded cDNA were prepared for subsequent PCR amplification. GAPDH mRNA was used as an internal quantitative control. Primer sequences used to amplify SNORA18, NUP85, WASF2, and SNORA22 are listed in Table 3 below.

**[Table 3]**

| | Forward | SEQ ID NO | Reverse | SEQ ID NO |
|---|---|---|---|---|
| SNORA18 | 5'- CCCTCTTGGTAGCTTCGTTC | 13 | 5'- CGCAGGTATGAAATAAGACTGAG | 14 |
| NUP85 | 5' - GCTCCTCCAGTCACACAACCT | 15 | 5'- CTCGGGGCAGTAATCAAAGTAATC | 16 |
| WASF2 | 5'- GTGCCAGCTTGGACAGATTGA | 17 | 5'- GGACACGGTGGGAATGCTTA | 18 |
| SNORA22 | 5'- GTGCAGGGAGAGGAATCAAT | 19 | 5'- GCATGTACGAAAGCTCCAGA | 20 |

PCR reactions were performed on a TaKaRa PCR Thermal Cycler Dice Gradient with initial denaturation at 94°C for 2 minutes, followed by 94°C for 30 seconds, 58°C for 30 seconds, and 72°C for 1 minute for 21 cycles (for GAPDH) or 25 cycles (for SNORA18, NUP85, WASF2, and SNORA22). Scanning and densitometric analysis for band intensity measurements were performed using the Quantity One analysis system (Bio-Rad).

As a result, as shown in Figures 4A, 5A, 6A, and 7A, the combination of control siRNA with Dab8, Fol-Dab8A, or Fol-Dab8B had no knockdown effect on SNORA18, NUP85, WASF2, and SNORA22, whereas remarkable knockdown effects were confirmed when SNORA18 siRNA, SNORA22 siRNA, NUP85 siRNA, and WASF2 siRNA were added in combination with Fol-Dab8A or Fol-Dab8B. This knockdown effect was not observed in Dab8 which did not contain folic acid.

### [Example 6: Cell Invasion Inhibitory Effect of Folic Acid-Cationic Oligopeptide Complex + siRNA]

Each of the scrambled control siRNA, SNORA18 siRNA, NUP85 siRNA, WASF2 siRNA, and SNORA22 siRNA (all used in Example 5) with the folic acid-cationic oligopeptide complex added was added to the culture solution of S2-013 cells, and Matrigel invasion assay was performed after 48 hours.

4.0 × 10⁴ cells were suspended in a serum-free medium and seeded in the upper chamber of a Matrigel Invasion Chamber (24-well plate, pore size: 8 µm, manufactured by Becton, Dickinson, and Company). A solvent containing 5% fetal bovine serum was added to the lower chamber. After the cells were incubated in the upper chamber for 20 hours, three independent areas were observed under a microscope, and the cells invading the lower chamber were counted. The same experiment was repeated three times, and the cell invasion ability of S2-013 cells into which each siRNA was taken up was compared.

Figures 4B, 5B, 6B, and 7B show the number of cells that have migrated from the upper chamber to the lower chamber in assays where scrambled control siRNA or any of SNORA18 siRNA, NUP85 siRNA, WASF2 siRNA, and SNORA22 siRNA was added to cells with a folic acid-cationic oligopeptide complex. The "^{∗}" denotes a significant difference at P < 0.05 as compared to the control in the t-test.

As a result, cell invasion was significantly suppressed in the S2-013 cells into which SNORA18 siRNA, NUP85 siRNA, WASF2 siRNA, or SNORA22 siRNA was incorporated, as compared with S2-013 cells into which control siRNA was incorporated.

From these results, it was confirmed that siRNA added in combination with a folic acid-cationic oligopeptide complex to a culture medium of cultured cells was taken up into S2-013 cells and inhibited the expression of snoRNA and mRNA involved in cell invasion.

### [Example 7: Effect on Chemically-modified siRNA]

Chemically-modified siRNA was prepared to increase stability, and the effects of folic acid-cationic oligopeptide complexes were investigated.

Table 4 shows the sequences of the sense strands and antisense strands of siRNAs used in this Example. Each contains chemically modified base(s) and a phosphorothioate bond at the 3' end.

**[Table 4]**

| | Sense strand | SEQ ID NO | Antisense strand | SEQ ID NO |
|---|---|---|---|---|
| SNORA18 | 5'- **U**ACUU**U**ACU**C**A**C**AGGAC**U**ATpsTps | 21 | 5'- **U**AGUCCUGUGAG**U**AAAG**U**AApsAps | 22 |
| NUP85 | 5'- GCGG**C**AGAUGACUGAA**C**AATpsTps | 23 | 5'- UUGUU**C**AGU**C**AUCUGCCGCUpsGpS | 24 |
| WASF2 | 5'- GGAU**U**AGAU**C**AU**U**AGCU**C**ATpsTps | 25 | 5'- UGAGC**U**AAUGAUC**U**AAUCC**U**psAps | 26 |
| SNORA22 | 5'- UGGCUUUGACCCUGUGC**U**ATpsTps | 27 | 5'- **U**AG**C**A**C**AGGGU**C**AAAGC**C**AApsGps | 28 |

| | | | | |
|---|---|---|---|---|
| ps = phosphorothioate modification Bold/underline = 2'-OMe modification | | | | |

The scrambled control siRNA (SEQ ID NOs: 3 and 4) and the above siRNAs were used to investigate uptake into cells in the same manner as in Example 4.

The siRNA (2.5 µg/mL) labeled with Alexa488 (Thermo Fisher Scientific Inc.) and 1, 2, or 3 equivalents of Dab8, Fol-Dab8A, and Fol-Dab8B were added to the culture solution of the pancreatic cancer cell line S2-013 (2 × 10⁴ cells/well) in culture.

Then, in the same manner as in Example 5, the effects of the chemically-modified siRNA on the invasion of pancreatic cancer cells were investigated in the presence or absence of the folic acid-cationic oligopeptide complex.

Each of the scrambled control siRNA (SEQ ID NOs: 3 and 4), SNORA18 siRNA (SEQ ID NOs: 21 and 22), NUP85 siRNA (SEQ ID NOs: 23 and 24), WASF2 siRNA (SEQ ID NOs: 25 and 26), and SNORA22 siRNA (SEQ ID NOs: 27 and 28) was added with 2 equivalents of Dab8, Fol-Dab8A, or Fol-Dab8B to the culture solution of pancreatic cancer cell line S2-013 in culture. As a control, each of the control siRNA, SNORA18 siRNA, NUP85 siRNA, WASF2 siRNA, and SNORA22 siRNA was added alone to the culture solution of the S2-013 cells.

After 48 hours of culture, the Matrigel invasion assay was performed. In the Matrigel invasion assay, the number of cells that have migrated from the upper chamber to the lower chamber is shown. The "^{∗}" denotes a significant difference at P < 0.05 as compared to the control in the t-test.

As a result, as shown in Figure 8, when SNORA18 siRNA, NUP85 siRNA, WASF2 siRNA, or SNORA22 siRNA was used in the presence of Fol-Dab8A or Fol-Dab8B, invasion of S2-013 cells was significantly suppressed as compared with S2-013 cells with scrambled control siRNA added in the presence of Fol-Dab8A and Fol-Dab8B, and with siRNA added alone. In the presence of folic acid-free Dab8, the suppressing effects of SNORA18 siRNA, NUP85 siRNA, WASF2 siRNA, or SNORA22 siRNA on S2-013 cell invasion were observed, but compared to the addition of Fol-Dab8A and Fol-Dab8B, the suppressing effects were weaker.

### [Example 8: Confirmation of Uptake of Chemically-Modified siRNA into Pancreatic Cancer Cells 1]

Chemically-modified SNORA22 siRNA (SEQ ID NOs: 27 and 28) prepared in Example 7 was labeled with Alexa488 (Thermo Fisher Scientific Inc.) and added with the folic acid-cationic oligopeptide complex (Fol-Dab8B, 2 equivalents) to the culture solution of S2-013 pancreatic cancer cells or HPNE normal pancreatic duct epithelial cells (ATCC) in a 4-well chamber (Thermo Fisher Scientific Inc.) (5 × 10⁴ cells/well). After incubation overnight at 37°C, cells were observed for staining of DNA (DAPI), folate receptors (FOLR1), and siRNA (Alexa488).

As a result, as shown in Figure 9, the staining intensity of the folate receptors was higher in the S2-013 pancreatic cancer cells than in the HPNE normal pancreatic duct epithelial cells, and at the same time, the staining intensity of SNORA22 siRNA taken up into the cells was stronger. This indicates that more SNORA22 siRNA is taken up by pancreatic cancer cells that express more folate receptors.

Figure 10 illustrates the incorporation efficiency (%) of SNORA22 siRNA into the S2-013 pancreatic cancer cells and the HPNE normal pancreatic duct epithelial cells in the presence of the folic acid-cationic oligopeptide complex (Fol-Dab8B, 2 equivalents). The uptake of the SNORA22 siRNA into the S2-013 pancreatic cancer cells was nearly 3-fold higher than that into normal cells.

### [Example 9: Confirmation of Uptake of Chemically-Modified siRNA into Pancreatic Cancer Cells 2]

It is known that siRNA taken up by endocytosis into cells is further taken up into endosomes and fused to lysosomes. To verify whether or not the complex of siRNA and Fol-Dab8A or Fol-Dab8B of the present invention is taken up via endocytosis, confocal microscope images of lysosome staining and siRNA staining were acquired.

Chemically-modified SNORA22 siRNA (SEQ ID NOs: 27 and 28) labeled with Alexa 488 and the folic acid-cationic oligopeptide complex (Fol-Dab8A or Fol-Dab8B, 2 equivalents) were added to the culture solution of S2-013 pancreatic cancer cells in a 4-well chamber (Thermo Fisher Scientific Inc.) (2 × 10⁴ cells/well), incubated overnight, and observed for staining of lysosomes (stained with LysoTracker, Thermo Fisher Scientific Inc.) and siRNA (Alexa488).

As a result, as shown in Figure 11, it was indicated that SNORA22 siRNA was taken up by the S2-013 pancreatic cancer cells and localized in lysosomes, suggesting that the siRNA and the folic acid-cationic oligopeptide complex were taken up by endocytosis.

### [Example 10: Confirmation of Improvement in Stability of siRNA by Chemical Modification]

In this Example, whether or not the stability of siRNA in serum was improved by chemical modification was investigated.

Chemically-unmodified SNORA22 siRNA (SEQ ID NOs: 11 and 12) or chemically-modified SNORA22 siRNA (SEQ ID NOs: 27 and 28) were mixed with a cationic oligopeptide (Dab8) or a folic acid-cationic oligopeptide complex (Fol-Dab8A or Fol-Dab8B) and allowed to stand at room temperature for 15 minutes, then added to PBS or 10% FCS/PBS in an amount of 1 µL, and mixed (final concentration: 20 µM). As a control, each SNORA22 siRNA was added alone.

Samples were taken immediately after mixing, after 3 hours, or after 6 hours, frozen in liquid nitrogen, and stored at -80°C. After all samples were obtained, SDS-PAGE using a non-reducing gel and detection with SYBR GOLD (Thermo Fisher Scientific Inc.) were performed.

As a result, as shown in Figure 12, siRNA without chemical modification is shown to be rapidly degraded even in the presence of the cationic oligopeptide or the folic acid-cationic oligopeptide complex. In contrast, it was suggested that chemical modification of siRNA improved the stability in serum and almost suppressed degradation by RNase.

### [Example 11: Uptake of siRNA In Vivo 1]

Human pancreatic cancer organoids were prepared using S2-013 pancreatic cancer cells by modifying the method described in JP Patent Application Publication No. 2018-110575 A1.

Specifically, the S2-013 pancreatic cancer cells (20 × 10⁴ cells), human mesenchymal stem cells MSC (LONZA, 40 × 10⁴ cells), and human umbilical vein endothelial cells HUVEC (LONZA, 14 × 10⁴ cells) were added to DMEM/Matrigel mixed solution in a 48-well plate (Thermo Fisher Scientific Inc.) and incubated in a CO₂ incubator at 37°C for 30 minutes.

Then, 300 µL/well of DMEM/EGM mixed solution was added thereto and incubated again at 37°C for 24 hours to prepare one pancreatic cancer organoid in each well.

Subsequently, the skin on the flank of nude mice (6-week-old BALB/cSlc-nu/nu (Pathogen-free female athymic nude mice), Japan SLC, Inc.) was incised, and the pancreatic cancer organoids obtained above were subcutaneously transplanted (2 mice in each group).

Six weeks after transplantation, the chemically-modified SNORA22 siRNA (SEQ ID NOs: 27 and 28, 5 µg) labeled with Alexa-594 was administered with 1, 2, or 3 equivalents of the cationic oligopeptide (Dab8) or the folic acid-cationic oligopeptide complex (Fol-Dab8A or Fol-Dab8B) by tail vein injection. After 24 hours, images were taken with an *in vivo* imager.

Measuring instrument: Spectrum *In Vivo* Imaging System (PerkinElmer, Inc., Waltham, MA)

Measurement condition: kexc 640nm, kemi 680nm

As a result, as shown in Figure 13, the delivery of SNORA22 siRNA to pancreatic cancer tissue was enhanced when Fol-Dab8A (D-F) and Fol-Dab8B (G-I) were used compared to when Dab8 was used (A-C). In particular, when 3 equivalents of Fol-Dab8A were added (F) and when 2 equivalents of Fol-Dab8B were added (H), the delivery was done at high concentration.

Note that in this experiment, the transfer to and accumulation in the liver of siRNA were hardly observed, and that the excretion of siRNA from the kidneys was also confirmed.

### [Example 12: Confirmation of Anti-Tumor Effect]

From 1 week after the human pancreatic cancer organoid prepared in Example 11 was subcutaneously transplanted into a nude mouse, the chemically-modified SNORA22 siRNA (SEQ ID NOs: 27 and 28, 5 µg) was administered once a week with the cationic oligopeptide (Dab8, 2 equivalents) or the folic acid-cationic oligopeptide complex (Fol-Dab8B, 2 equivalents) by tail vein injection, and tumor volume was measured weekly using a caliper (n = 8 in each group). As a control, scrambled control siRNA (SEQ ID NOs: 3 and 4) was administered with the folic acid-cationic oligopeptide complex (Fol-Dab8B, 2 equivalents).

As a result, as shown in Figure 14, the results of measurements up to 9 weeks after the initiation of administration revealed that, compared to the non-administered control group (Control), the group with the scrambled control siRNA administration (Scr-Fol-Dab8B), and the group administered with the cationic oligopeptide (SNORA22-Dab8), significant suppressing effects on tumor growth were observed in the group in which SNORA22 siRNA was administered together with the folic acid-cationic oligopeptide complex (SNORA22-Fol-Dab8B) after 8 weeks and later.

### [Example 13: Uptake of siRNA In Vivo 2]

In the same manner as in Example 11, the delivery of chemically-modified SNORA18 siRNA (SEQ ID NOs: 21 and 22) to pancreatic cancer-carrying mice when administered with the folic acid-cationic oligopeptide complex (Fol-Dab8A or Fol-Dab8B) was imaged using an *in vivo* imager.

As a result, as shown in Figure 15, it was confirmed that both Fol-Dab8A and Fol-Dab8B enhanced the delivery of SNORA18 siRNA to pancreatic cancer tissue. In particular, the delivery at high concentration was achieved when Fol-Dab8A was added in an amount of 1 equivalent (A) or 3 equivalents (C).

### [Example 14: Uptake of siRNA In Vivo 3]

In the same manner as in Examples 11 and 13, the delivery of chemically-modified WASF2 siRNA (SEQ ID NOs: 25 and 26) to pancreatic cancer-carrying mice when administered with the folic acid-cationic oligopeptide complex (Fol-Dab8A or Fol-Dab8B) was imaged using an *in vivo* imager.

As a result, as shown in Figure 16, it was confirmed that both Fol-Dab8A and Fol-Dab8B enhanced the delivery of WASF2 siRNA to pancreatic cancer tissue. In particular, the delivery at high concentration was achieved when Fol-Dab8B was added in an amount of 1 equivalent (D) or 3 equivalents (F).

### Industrial Applicability

The present invention provides therapeutic measures capable of effectively suppressing tumor growth, invasion, and metastasis of pancreatic cancer, which is said to have the worst prognosis among cancers. The anti-tumor agent of the present application is effectively delivered specifically to pancreatic cancer cells and, when combined with other anti-cancer agents and/or anti-cancer therapies, can dramatically enhance the therapeutic effect on pancreatic cancer.

All publications, patents, and patent applications cited herein are incorporated herein by reference in their entirety.

## Claims

1. An anti-tumor agent comprising siRNA or shRNA capable of binding to mRNA or snoRNA expressed in pancreatic cancer cells to inhibit its expression, and a delivery enhancer of siRNA or shRNA consisting of a folic acid-cationic oligopeptide complex, wherein the cationic oligopeptide in the complex comprises a cationic oligopeptide moiety consisting of 8 to 40 amino acids, comprising at least two contiguous amino acid residues of the following formula (I), and optionally comprising another non-contiguous amino acid residue other than the contiguous amino acid residues of the following formula (I): 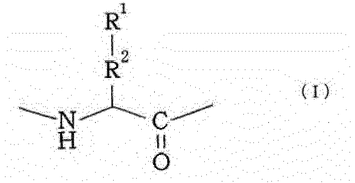 wherein R¹ is a group H₃N⁺-CH₂- or a group represented by formula (II); R² is absent or an alkylene group having 1 to 3 carbon atoms when R¹ is the group H₃N⁺-CH₂-, or R² is an alkylene group having 1 to 4 carbon atoms when R¹ is a group represented by formula (II); and in one cationic oligopeptide, all R¹ are the same and all R² are the same: 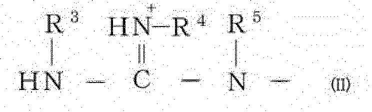 wherein R³, R⁴, and R⁵ are the same or different and are each a hydrogen atom or a methyl group.

2. A delivery enhancer of siRNA or shRNA consisting of a folic acid-cationic oligopeptide complex, wherein the cationic oligopeptide comprises a cationic oligopeptide moiety consisting of 8 to 40 amino acids, comprising at least two contiguous amino acid residues of the following formula (I), and optionally comprising another non-contiguous amino acid residue other than the contiguous amino acid residues of the following formula (I): 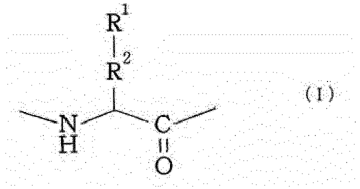 wherein R¹ is a group H₃N⁺-CH₂- or a group represented by formula (II); R² is absent or an alkylene group having 1 to 3 carbon atoms when R¹ is the group H₃N⁺-CH₂-, or R² is an alkylene group having 1 to 4 carbon atoms when R¹ is a group represented by formula (II); and in one cationic oligopeptide, all R¹ are the same and all R² are the same: 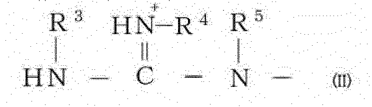 wherein R³, R⁴, and R⁵ are the same or different and are each a hydrogen atom or a methyl group.

3. The delivery enhancer according to claim 2, wherein the cationic oligopeptide moiety consists of 8 to 12 amino acids.

4. The delivery enhancer according to claim 2 or 3, wherein the cationic oligopeptide moiety is a homomultimer of L-2,3-diaminopropionic acid (Dap), L-2,4-diaminobutyric acid (Dab), L-ornithine (Orn), L-lysine (Lys), L-2-amino-3-guanidinopropionic acid (Agp), L-2-amino-4-guanidinobutyric acid (Agb), or L-arginine (Arg).

5. The delivery enhancer according to any one of claims 2 to 4, wherein the cationic oligopeptide has as substructure an octamer of diaminobutyric acid having the following structure.

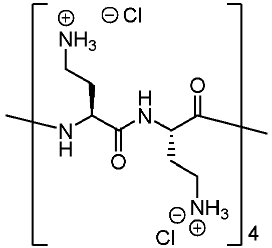

6. The delivery enhancer according to any one of claims 2 to 5, wherein the folic acid is linked to the N-terminus, the C-terminus, or a side chain of the cationic oligopeptide via a linker or no linker.

7. The delivery enhancer according to claim 6, wherein the folic acid-cationic oligopeptide complex is linked via a linker, the linker being a peptide linker.

8. The delivery enhancer according to claim 7, wherein the peptide linker is a peptide consisting of 1 to 4 glycine residues.

9. The delivery enhancer according to claim 5, wherein the folic acid-cationic oligopeptide complex is Fol-Dab8A and/or Fol-Dab8B having the following structure.

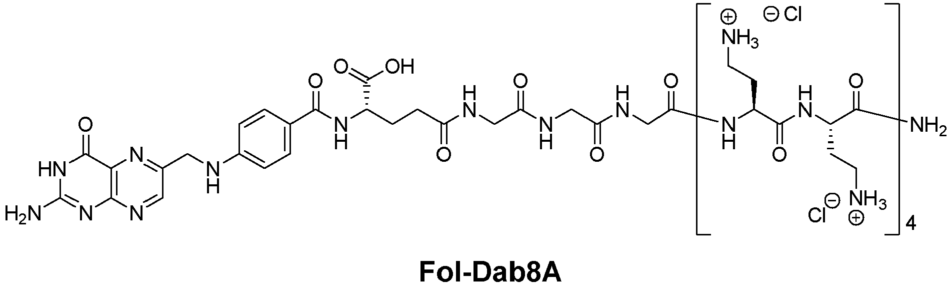

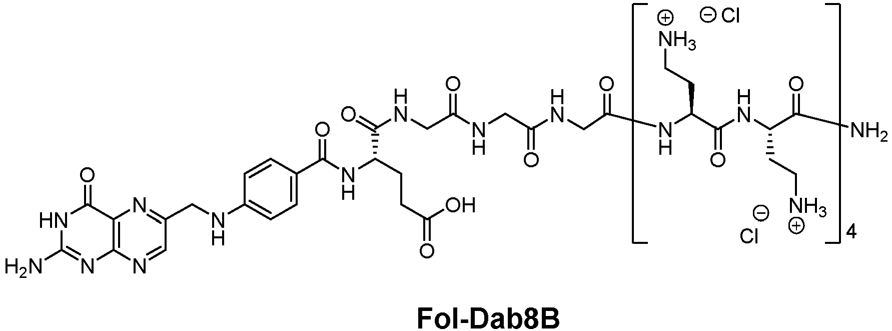

10. An anti-tumor agent comprising: siRNA or shRNA capable of binding to mRNA or snoRNA expressed in pancreatic cancer cells to inhibit its expression; and the delivery enhancer according to any one of claims 2 to 9.

11. The anti-tumor agent according to claim 10, wherein the mRNA or snoRNA expressed in pancreatic cancer cells binds to insulin-like growth factor 2 mRNA-binding protein 3 (IGF2BP3).

12. The anti-tumor agent according to claim 10 or 11, wherein the mRNA or snoRNA expressed in pancreatic cancer cells is selected from the group consisting of SNORA18 snoRNA, NUP85 mRNA, WASF2 mRNA, and SNORA22 snoRNA.

13. The anti-tumor agent according to any one of claims 10 to 12, comprising 0.5 to 10 equivalents of the folic acid-cationic oligopeptide complex relative to the siRNA or shRNA.

14. The anti-tumor agent according to any one of claims 10 to 13, wherein the siRNA is an RNA-RNA duplex.

15. The anti-tumor agent according to any one of claims 10 to 14, wherein the siRNA or shRNA comprises a modified base, a modified sugar, and/or an altered internucleoside bond.

16. The anti-tumor agent according to claim 15, wherein the modification of the modified sugar is 2'-OMe modification.

17. The anti-tumor agent according to claim 15 or 16, wherein the altered internucleoside bond is phosphorothioate bond.

18. A pharmaceutical composition comprising the anti-tumor agent according to any one of claims 10 to 17.

19. A combined formulation comprising:
(a) a formulation comprising siRNA or shRNA capable of binding to mRNA or snoRNA expressed in pancreatic cancer cells to inhibit its expression; and
(b) a formulation comprising a folic acid-cationic oligopeptide complex comprising a cationic oligopeptide moiety consisting of 8 to 40 amino acids, comprising at least two contiguous amino acid residues of the following formula (I), and optionally comprising another non-contiguous amino acid residue other than the contiguous amino acid residues of the following formula (I): 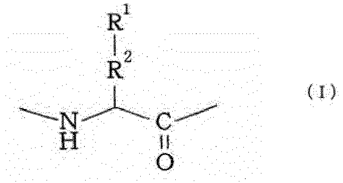
wherein R¹ is a group H₃N⁺-CH₂- or a group represented by formula (II); R² is absent or an alkylene group having 1 to 3 carbon atoms when R¹ is the group H₃N⁺-CH₂- or R² is an alkylene group having 1 to 4 carbon atoms when R¹ is a group represented by formula (II); and in one cationic oligopeptide, all R¹ are the same and all R² are the same: 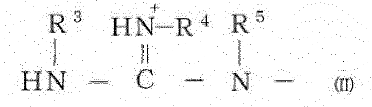 wherein R³, R⁴, and R⁵ are the same or different and are each a hydrogen atom or a methyl group.

20. A pharmaceutical kit for treating pancreatic cancer comprising:
(a) siRNA or shRNA capable of binding to mRNA or snoRNA expressed in pancreatic cancer cells to inhibit its expression; and
(b) a folic acid-cationic oligopeptide complex comprising a cationic oligopeptide moiety consisting of 8 to 40 amino acids, comprising at least two contiguous amino acid residues of the following formula (I), and optionally comprising another non-contiguous amino acid residue other than the contiguous amino acid residues of the following formula (I): 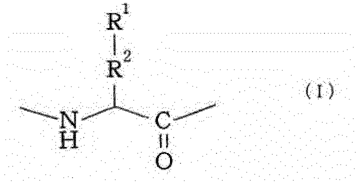
wherein R¹ is a group H₃N⁺-CH₂- or a group represented by formula (II); R² is absent or an alkylene group having 1 to 3 carbon atoms when R¹ is the group H₃N⁺-CH₂- or R² is an alkylene group having 1 to 4 carbon atoms when R¹ is a group represented by formula (II); and in one cationic oligopeptide, all R¹ are the same and all R² are the same: 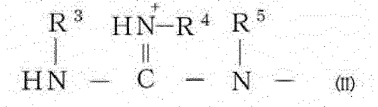 wherein R³, R⁴, and R⁵ are the same or different and are each a hydrogen atom or a methyl group.
